# EUROPEAN PATENT SPECIFICATION

(11) **EP 3 559 215 B1**
(45) Date of publication and mention of the grant of the patent: **24.08.2022**
(21) Application number: 17823197.3
(22) Date of filing: 22.12.2017
(51) Int. Cl.: C12N 5/0783, C12N 15/63, C07K 14/725

(54) **T CELL-TARGETED T CELLS**
GEGEN T-ZELLEN GERICHTETE T-ZELLEN
CELLULES T CIBLÉES PAR DES CELLULES T

(30) Priority: 22.12.2016 GB 201622044
(43) Date of publication of application: 30.10.2019
(73) Proprietor: UCL Business Ltd, London WC1E 6BT (GB)
(72) Inventor: QASIM, Waseem, Woking Surrey GU21 7PN (GB); MOCK, Ulrike, London W1T 4TP (GB); RASAIYAAH, Jane, London Greater London N19 5PW (GB); PREECE, Roland, London W1T 4TP (GB); GEORGIADIS, Christos, London W1T 4TP (GB)
(74) Representative: J A Kemp LLP
(86) International application number: PCT/GB2017/053888
(87) International publication number: WO 2018/115906

(56) References cited:
- WO-A1-2016/069282
- WO-A1-2016/138491
- WO-A1-2016/172606
- WO-A1-2018/027036
- WO-A1-2018/115887
- DIEGO SILVA ET AL: "CD7 CAR for the Treatment of Acute Myeloid and Lymphoid Leukemia", BLOOD 128, 1 December 2016 (2016-12-01), pages 4555-4555, XP055452712,
- L. POIROT ET AL: "Multiplex Genome-Edited T-cell Manufacturing Platform for "Off-the-Shelf" Adoptive T-cell Immunotherapies", CANCER RESEARCH, vol. 75, no. 18, 16 July 2015 (2015-07-16) , pages 3853-3864, XP055221500, US ISSN: 0008-5472, DOI: 10.1158/0008-5472.CAN-14-3321
- H. TORIKAI ET AL: "A foundation for universal T-cell based immunotherapy: T cells engineered to express a CD19-specific chimeric-antigen-receptor and eliminate expression of endogenous TCR", BLOOD, vol. 119, no. 24, 14 June 2012 (2012-06-14), pages 5697-5705, XP055071623, ISSN: 0006-4971, DOI: 10.1182/blood-2012-01-405365
- M. MAMONKIN ET AL: "A T-cell-directed chimeric antigen receptor for the selective treatment of T-cell malignancies", BLOOD, vol. 126, no. 8, 20 August 2015 (2015-08-20), pages 983-992, XP055325148, US ISSN: 0006-4971, DOI: 10.1182/blood-2015-02-629527
- KEVIN H. CHEN ET AL: "Novel anti-CD3 chimeric antigen receptor targeting of aggressive T cell malignancies", ONCOTARGET, vol. 7, no. 35, 30 August 2016 (2016-08-30), XP055452924, DOI: 10.18632/oncotarget.11019
- DIOGO GOMES-SILVA ET AL: "CD7-edited T cells expressing a CD7-specific CAR for the therapy of T-cell malignancies", BLOOD, vol. 130, no. 3, 24 May 2017 (2017-05-24), pages 285-296, XP055452686, US ISSN: 0006-4971, DOI: 10.1182/blood-2017-01-761320
- YI TIAN PNG ET AL: "Blockade of CD7 expression in T cells for effective chimeric antigen receptor targeting of T-cell malignancies", BLOOD ADVANCES, vol. 1, no. 25, 28 November 2017 (2017-11-28), pages 2348-2360, XP055452690, ISSN: 2473-9529, DOI: 10.1182/bloodadvances.2017009928
- CHRISTOS GEORGIADIS ET AL: "Emerging applications of gene edited T cells for the treatment of leukemia", EXPERT REVIEW OF HEMATOLOGY, vol. 10, no. 9, 25 July 2017 (2017-07-25), pages 753-755, XP055452698, UK ISSN: 1747-4086, DOI: 10.1080/17474086.2017.1350575
- Anonymous: "T-cell chimeric antigen receptor therapy for T-cell leukaemia*", , 1 January 2017 (2017-01-01), XP055452696, Retrieved from the Internet: URL:http://www.ucl.ac.uk/ich/education/fel lowships-studentships/phd-studentships/chr -phdstudentships-2018-19/w-qasim [retrieved on 2018-02-21]

## Description

### Field of the invention

The invention relates to T cell-targeted T cells, and methods employed in their production.

### Background to the invention

Cell therapy is therapy in which cellular material is introduced into a patient. Generally, intact, living cells are introduced to the patient. For example, T cells targeting disease-causing cells, such as cancer cells or immune cells, may be introduced to a patient. In this way, cancer cells may be destroyed or host immunity ablated.

Therapeutic T cells may comprise modifications associated with their therapeutic effect. For instance, a therapeutic cell may be modified to be targeted towards an antigen of interest, or to express a particular therapeutic molecule. T cells express heterodimeric antigen specific T cell receptors comprising disulphide bridged α and β chains or γ and δ chains in association with a multimeric CD3 complex. Exogenous molecules (e.g. an antigen receptor or therapeutic molecule) can be introduced to a therapeutic cell by transfecting or transducing the therapeutic cell with a nucleic acid sequence or construct encoding the molecule (i.e. a transgene). Such transfection and transduction is well known in the art.

In this way, therapeutic T cells may be engineered to direct their cytotoxic effects towards a particular antigen of interest. For example, T cells for killing tumour cells may be specific for a tumour antigen. T cell specificity may directed by endogenous αβ T cell receptors, or via introduced recombinant αβ receptors or by chimeric antigen receptors (CARs). The latter usually incorporates a single chain variable fragment (scfv) derived from the antigen binding regions of an antibody, linked to transmembrane and intracellular activation domains.

Recently, T cells transduced to express a CAR specific for CD19 (CAR19 T cells) have reached the clinic. These T cells have been shown to effectively target B cell malignancies expressing CD19. So far, though, it has not been possible to use CAR-expressing T cells therapeutically to effectively target T cell malignancies or unwanted T cells. One of the major problems in this respect is "T on T" cytotoxicity, where T cell-targeted therapeutic T cells target other such cells. Because T cell-targeted therapeutic T cells recognise and attack any cell expressing the T cell antigen for which they are specific, they recognise and attack desirable, therapeutic T cells as well as the malignant or unwanted T cells they are designed to ablate. In other words, T cell-targeted T cells have traditionally been subject to fratricide i.e. killing by other T cell-targeted therapeutic T cells. Thus, during manufacture and also presumably if T cell-targeted T cells were administered to a patient, the engineered cells attack each other. This depletes the population and its associated therapeutic effect.

Accordingly, there exists a need for T cell-targeted T cells that are not subject to fratricide. Silva et al. (Blood 128, 1 December 2016, page 4555) describes the generation of CD7-CAR-T cells following genomic disruption of the CD7 gene. WO 2016/138491 describes chimeric antigen receptor polypeptides having antigen recognition domains for CD5 or CD7 antigen, polypetides encoding the same, and engineered cells expressing the polypeptides.

### Summary of the disclosure

The present disclosure aims to provide a T cell that expresses a CAR specific for a CD3, but which is not itself targeted by T cells specific for CD3. Such a T cell-targeted T cell may be use therapeutically to deplete malignant or unwanted T cells, for instance to treat cancer or ablate host immunity. In addition, the T cell-targeted T cell has improved longevity, because it is not recognised and attacked by other T cell-targeted T cells. Thus, the T cell-targeted T cell of the invention has a reduced susceptibility to fratricide.

In more detail, the present inventors have found that a T cell-targeted T cell's susceptibility to fratricide can be reduced by disrupting the endogenous expression of one or more T cell receptor (TCR)/CD3 complex components in the T cell. When the expression of a TCR complex component is disrupted in the T cell, the T cell becomes less visible to T cells expressing an antigen receptor (such as a CAR or a TCR) specific for a TCR/CD3 complex componen. Therefore, the T cell-targeted T cell is less able to be targeted by other T cell-specific T cells.

The disclosure also relates to a highly efficient method for generating such T cell-targeted T cells. The method of the invention provides a surprisingly high yield of T cell-targeted T cells that resist fratricide. The population of T cell-targeted T cells is surprisingly pure. When T cell-targeted T cells of the invention are produced according to the new method, any residual T cell-targeted T cells that remain visible to T cells expressing an antigen receptor specific for a TCR/CD3 complex componentare attacked by the other T-cell targeted T cells in the population. Thus, T cell-targeted T cells of the invention whose endogenous TCR/CD3 complex expression is disrupted acquire a survival advantage, and these populations are enriched or 'self-selected' in culture as they lyse non-modified T cells. This means that further enrichment steps (such as antibody-mediated selection of transduced cells or depletion of residual TCR/CD3 - expressing cells, by fluorescence activated cell sorting or magnetic activated cell sorting) are not required.

### Summary of the invention

The invention is defined in claim 1. In particular, the invention provides a method for producing a T cell expressing one or more chimeric antigen receptors (CARs) specific for CD3, comprising (i) eliminating endogenous expression of a T cell receptor (TCR)/CD3 complex component in a T cell, (ii) introducing a nucleic acid sequence encoding the one or more CARs to said T cell of (i), and subsequently (iii) expressing the one or more CARs of (ii) in said T cell of (i); wherein (i) is carried out 10 to 40 hours before (ii).

Further aspects and preferred embodiments are defined in the dependent claims. Any aspects, embodiments and examples of the present disclosure which do not fall under the scope of the appended claims do not form part of the invention and are merely provided for illustrative purposes.

### Brief Description of the Figures

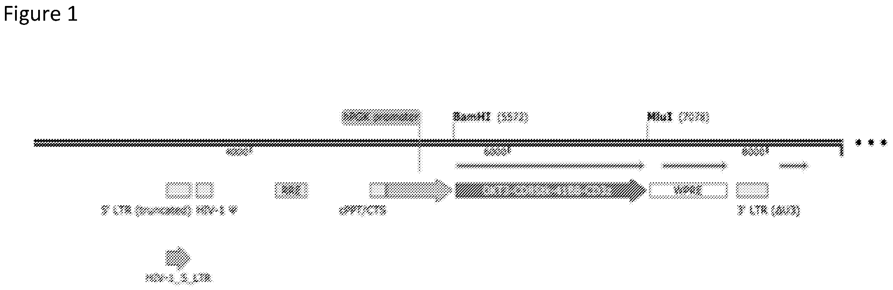

Figure 1: 3CAR plasmid construct.

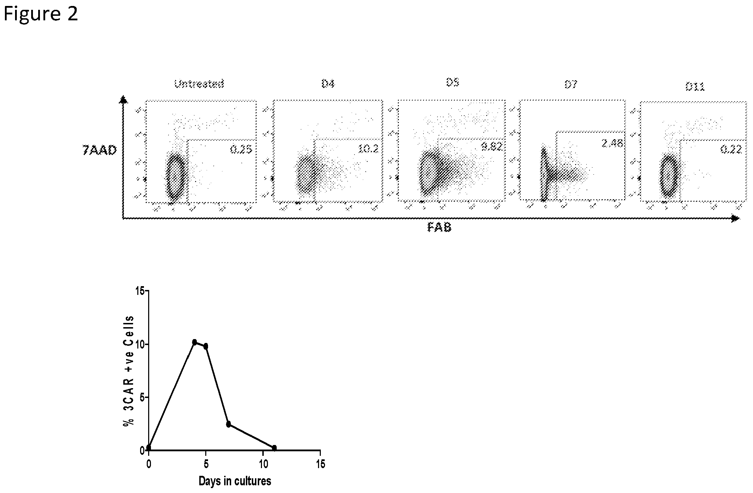

Figure 2: 3CAR expressing human T cells do not propagate in culture following LV transduction of PBMNC.

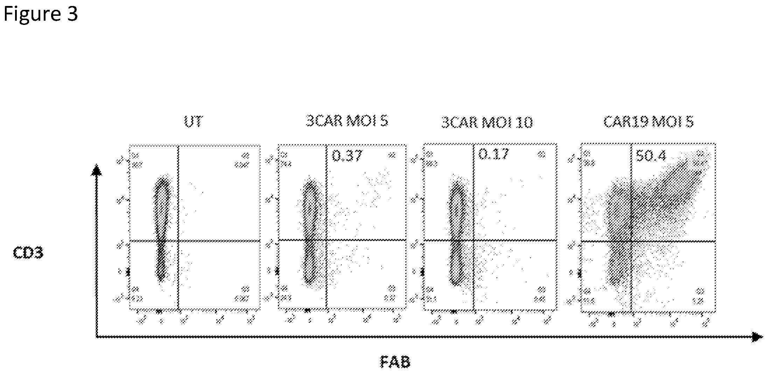

Figure 3: Reduced 3CAR propagation is specific to 3CAR, and does not affect LV CAR19 cells

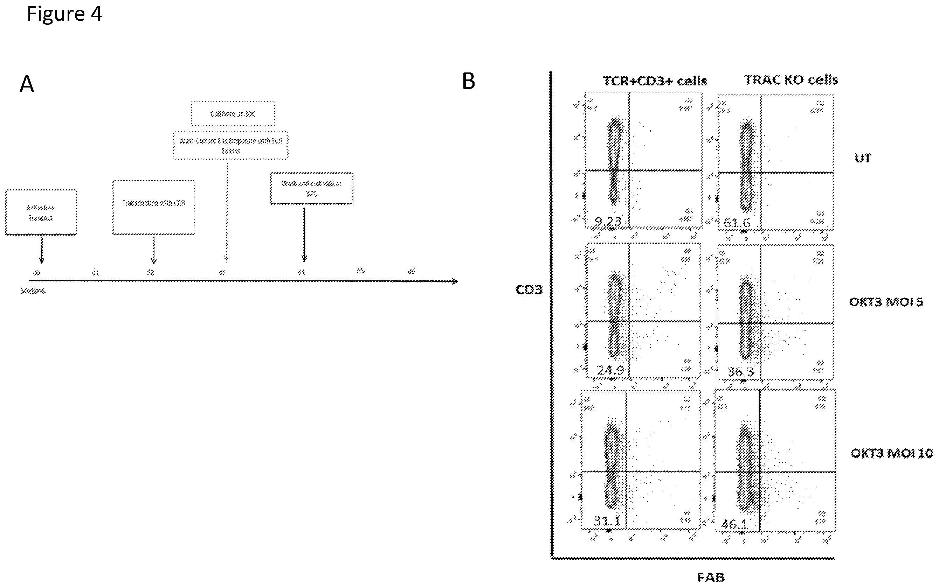

Figure 4: Conventional process with LV-CAR transduction followed by TRAC ko by TALENs did not produce 3CAR yields

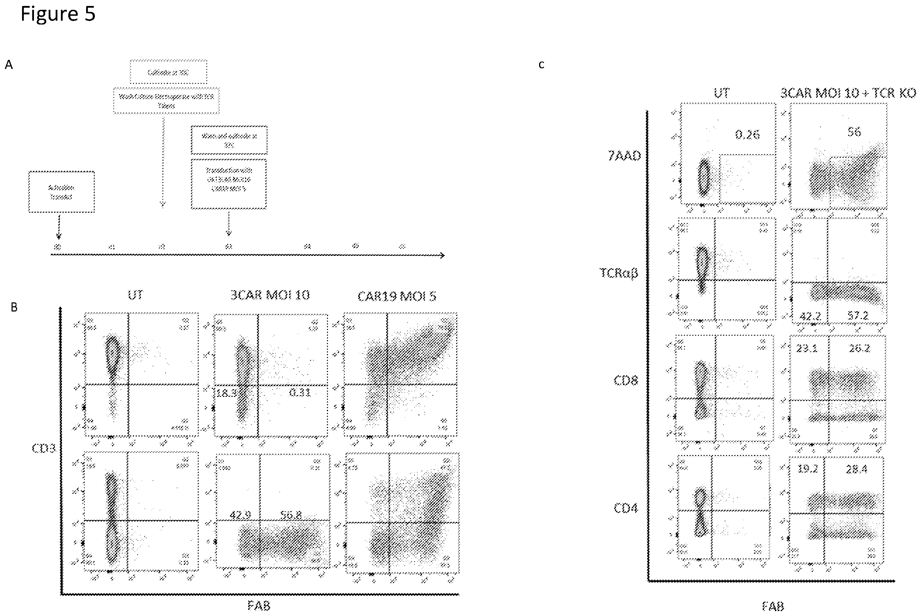

Figure 5: High 3CAR yields produced by TRAC ko 24 hours prior to LV3CAR transduction

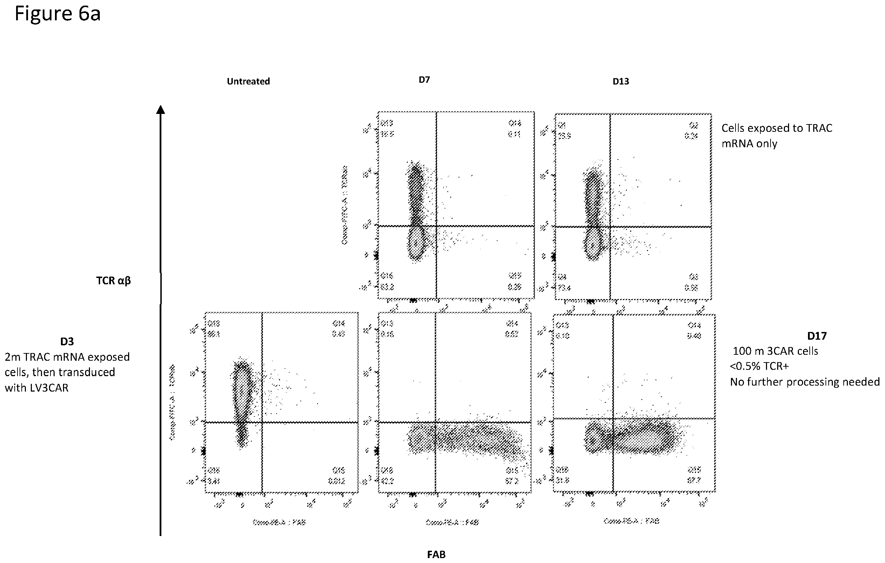

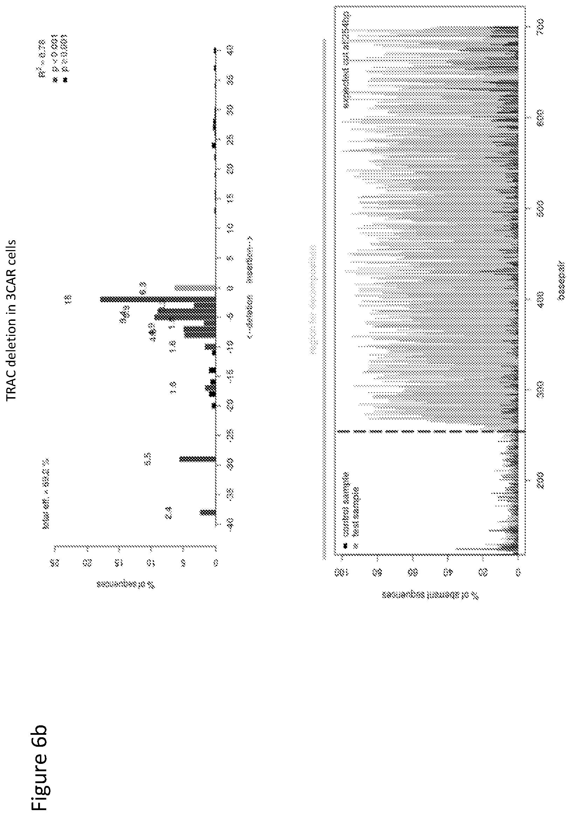

Figure 6: High transduction efficiency and sustained expression of 3CAR T cells require no further processing

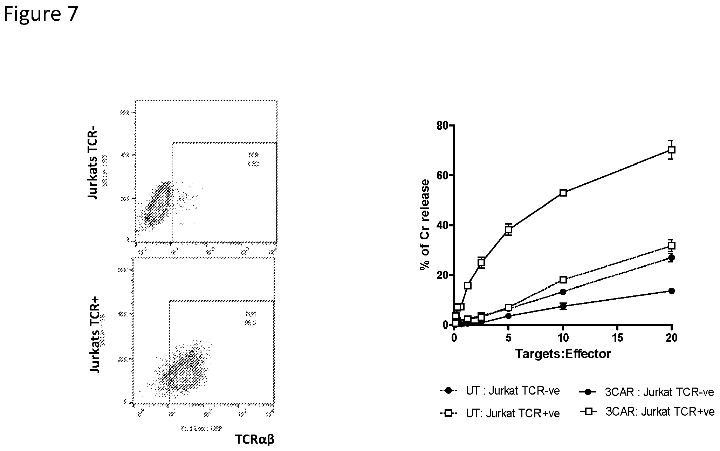

Figure 7: High transduction efficiency and sustained expression of 3CAR T cells capable of effector function against CD3 leukemic cells

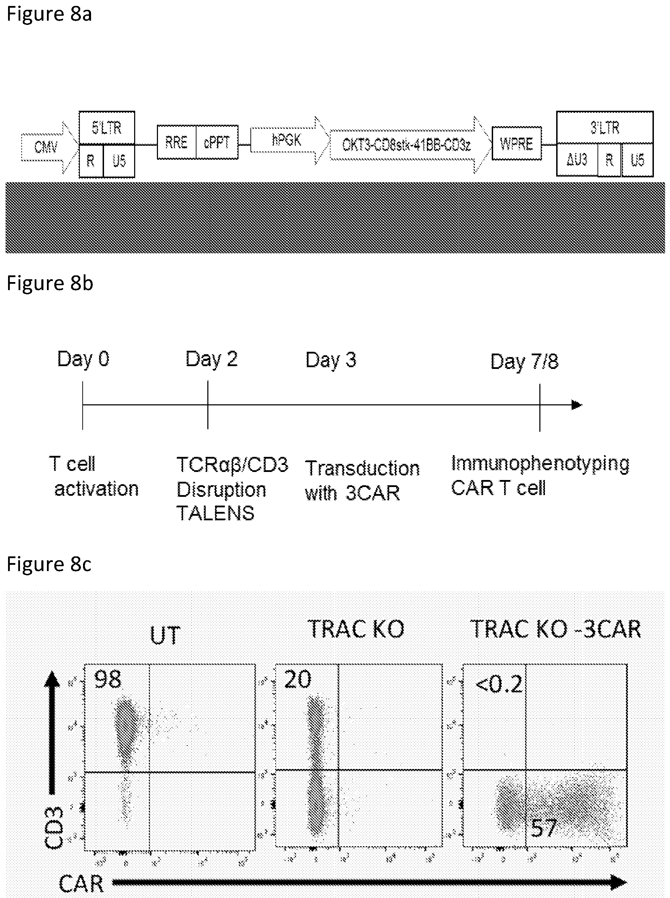

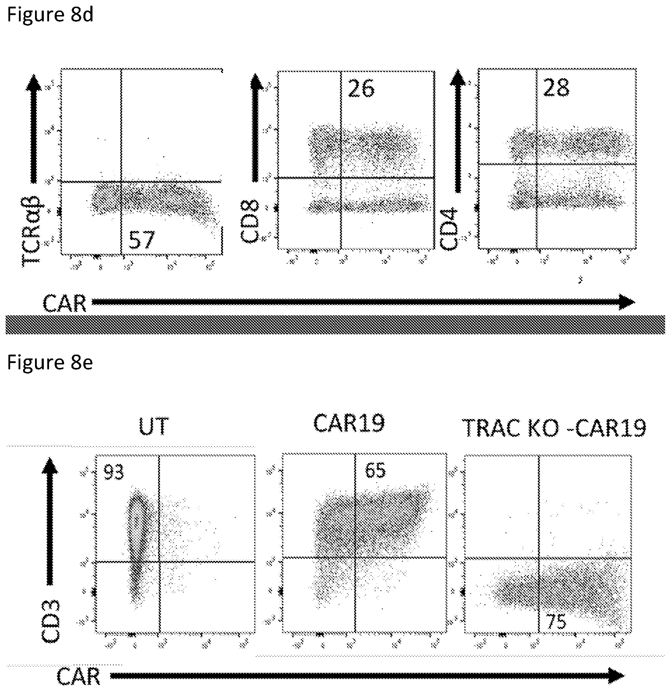

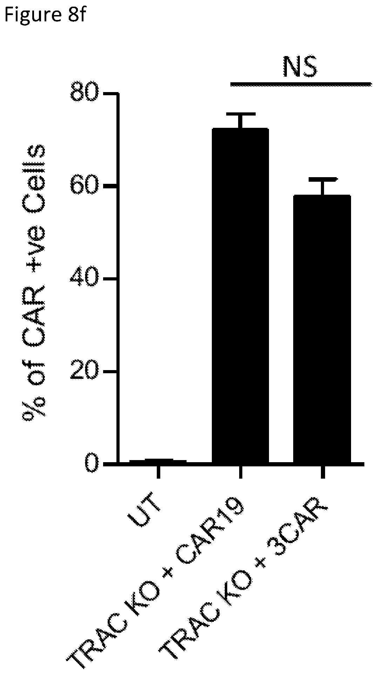

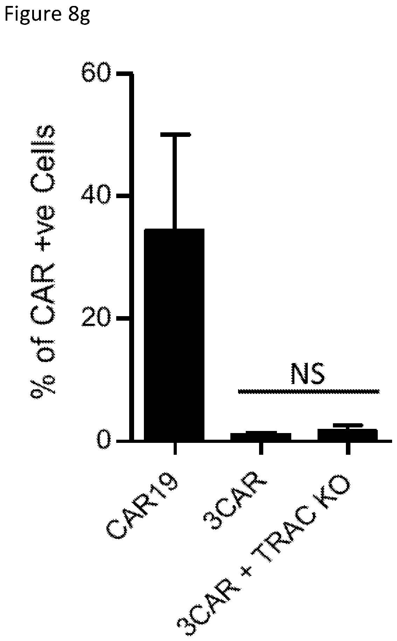

Figure 8: Successful generation of 3CAR requires disruption of CD3e expression prior to LV3CAR transduction. A) Vector configuration showing codon optmised scFV derived from OKT3 with CD8 stalk and 41BB/CD3z activation domains, all under the control of an internal hPGK promoter in a third generation sin-lentivirus with HIV-1 derived RRE, cPPT and mutated WPRE elements B) Schema of event precedence required for successful 3CAR T cell production. CD3/CD28 activation with Transact for 48hours was followed by electroporation of mRNA encoding TRAC specific TALENs ahead of LV transduction by 72 hours. C) After 7 days cells were analysedby flow cytometry and in cultures with 3CAR expression there were no surviving CD3+ cells compared to 20% residual expression in the absence of 3CAR transduction. D) 3CAR T cells retained CD4 or CD8 but not TCRab expression. E) For comparison, control transductions with LVCAR19mediated 65% transduction but required further processing by column mediated TCRab depletion to yield TRAC^{ko}CAR19+ T cells. F) Summary of TRAC^{ko} productions from three independent experiments. G) Summary of CAR+ cells with and without TRAC^{ko} prior to LV from three independent experimentswith 3 donors. P-value NS by unpaired, 2-tailed Student's t test.

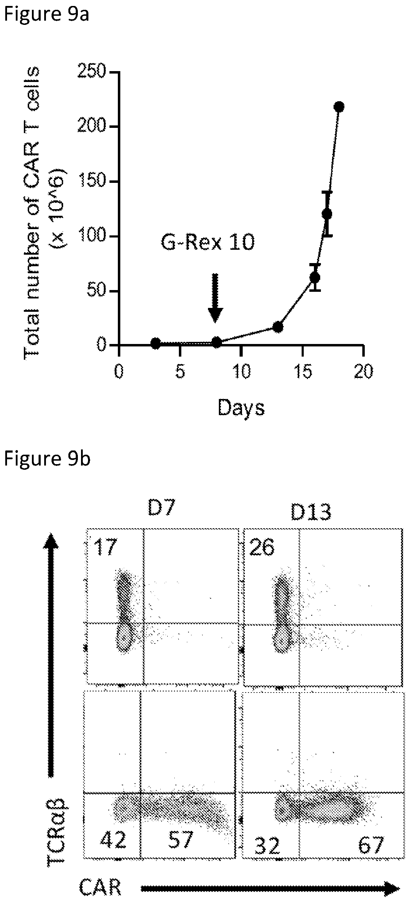

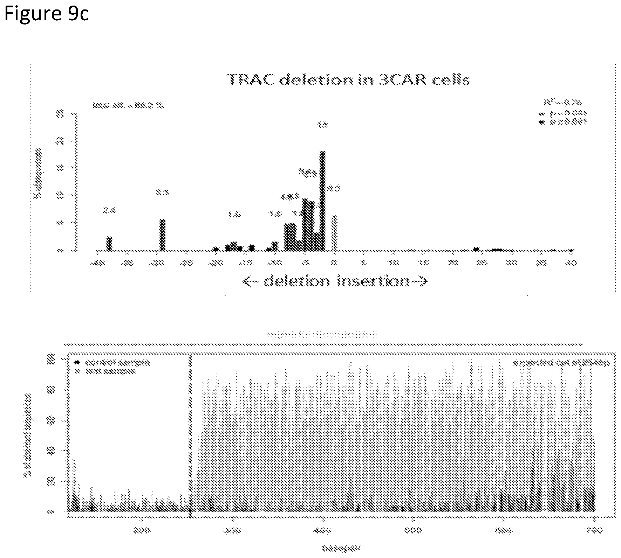

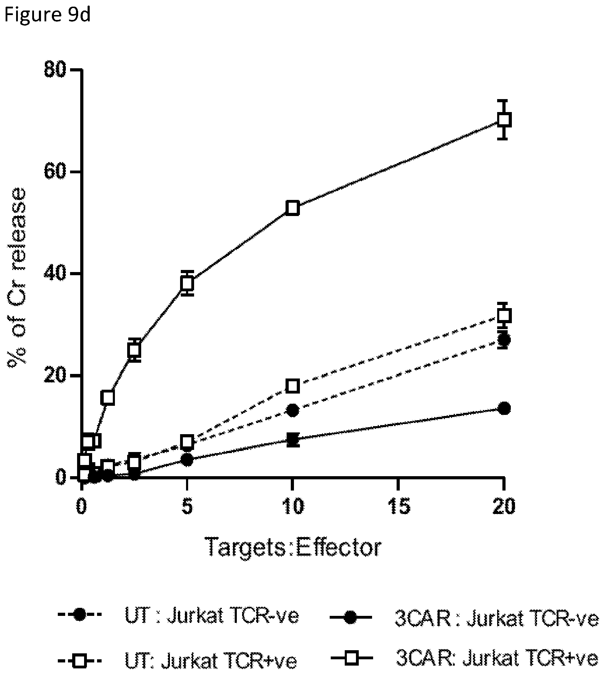

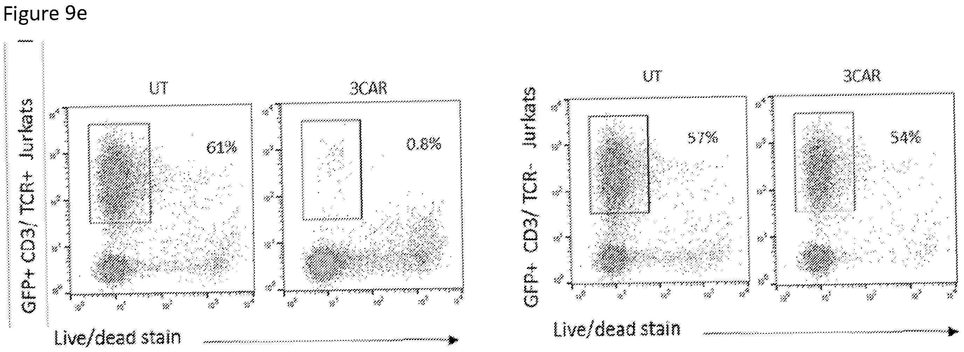

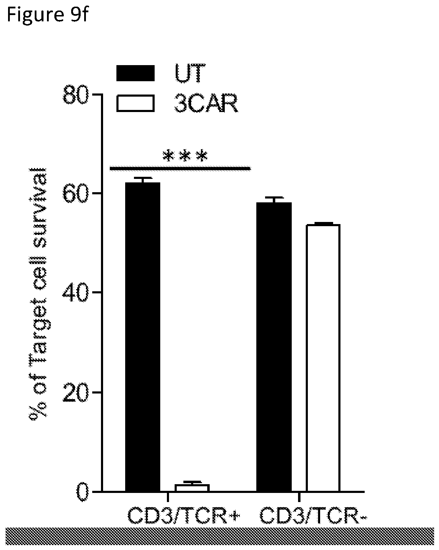

Figure 9: 3CAR T cells self-enrich and mediate potent leukemia killing in vitro. A)Primary T cells were activated, electroporated with TALEN TRACmRNA exposure and then transduced and expanded in a G-Rex flask over 17days B) Expression of CD3/TCR on day 7 and 13 confirming absence of CD3+ cells in lower panels after 3CAR lentiviral expression. C) TIDE-PCR confirmed 70%of alleles harboured molecular signatures of non homologous end joining across the TRAC locus. D) 51Cr labelled CD3+TCR+ve (white symbols) or CD3-TCR-ve (black symbols) Jurkat leukaemia cells were cocultured with either 3CAR T cells (solid lines) or Untransduced controls (dotted lines). e) 3CAR T cells were cocultured with either GFP+CD3+/TCR+ or GPP+CD3-TCR- Jurkat leukaemia cells at a effector:target ratio of 1:1 for 24hr. Representative flow cytometry plots of gated on GFP+ leukemia cells at the end of coculture demonstrate target specific killing. F) Summary of 3CAR cytotoxicity in three independent experiments with 3 donors, error bars=SEM. ^{∗∗∗}P<0.0005, by unpaired, 2-tailedStudent's t test.

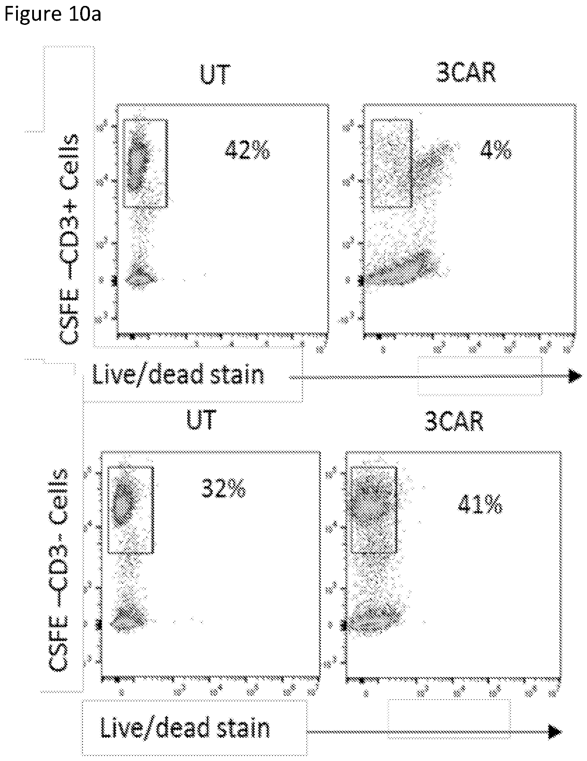

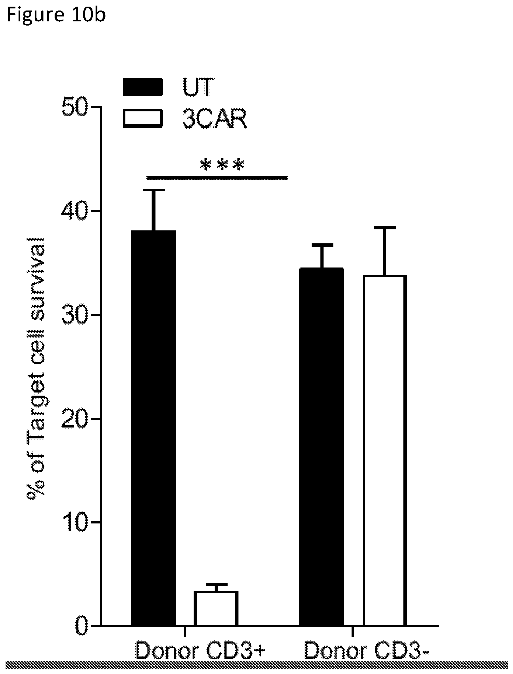

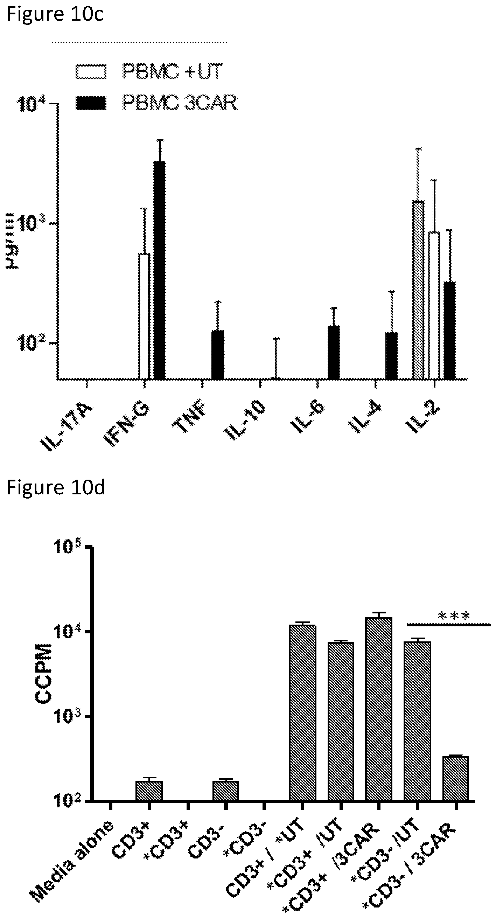

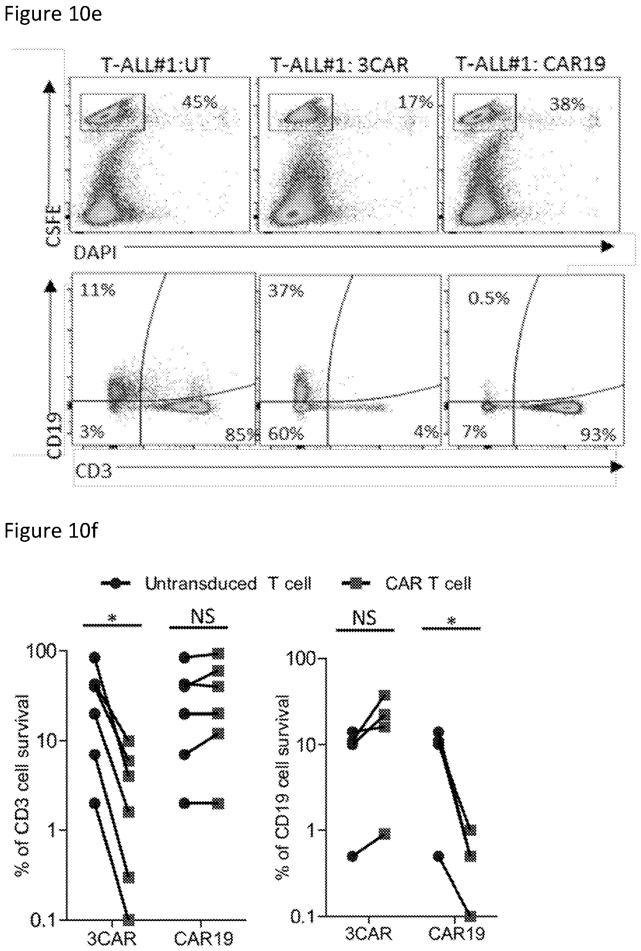

Figure 10: 3CAR T cells specifically targets CD3 in primary healthy donor T cells and childhood T-ALL cells. A) andBD) 3CAR or untransduced T cells were cocultured with either CSFE loaded primary CD3+ and CD3- cells at the effector target ratio of 1:1 for 24hr. C) Representative flow cytometry plots of gated CSFE+ tumour cells at the end of coculture. D) Summary of three independent experiments with 3 donors, error bars=SEM. ^{∗∗∗}P < 0.0005, by unpaired, 2-tailed Student's t test. E) Cytokine production by 3CAR T cells when co-cultured with healthy donor PBMC target cells were comparble to untransduced cells. Data represent two independent experiments, and 3 donors. F) Mixed lymphocyte reactions of 3CAR or untransduced T cells cultured with irradiated (^{∗}) allogeneic PBMC or CD3- cells. One representative of 2 independent experiments captures 3H thymidine proliferation responses consistent with specific targeting of CD3 rather than alloreactive proliferation.. ^{∗}P< 0.05, by unpaired, 2-tailed Student's t test.

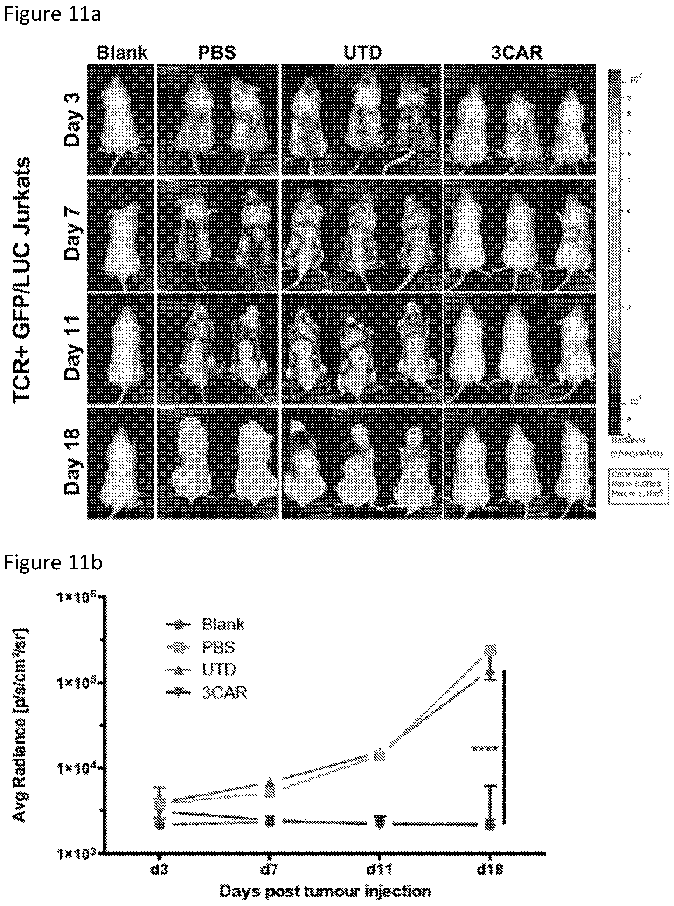

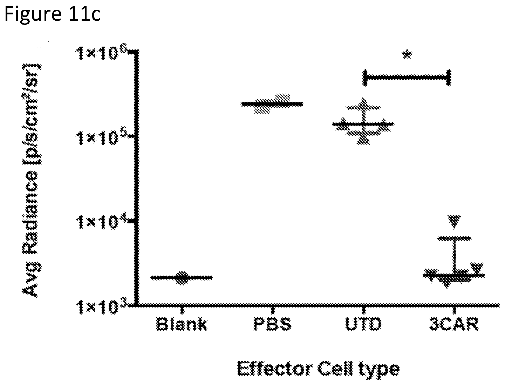

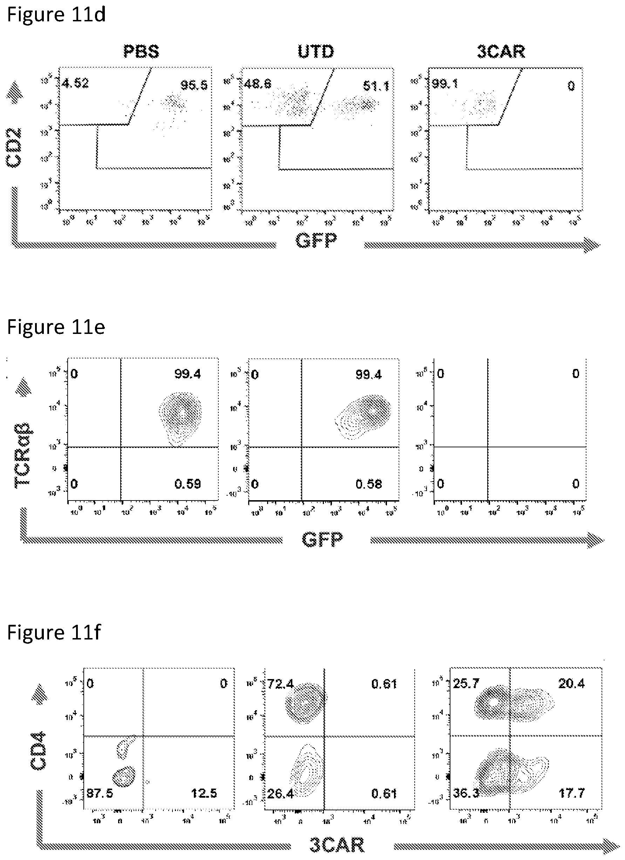

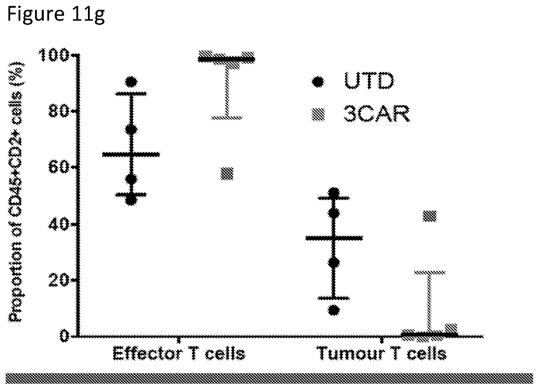

Figure 11: Anti-leukeamic responses by 3CAR effector T cells against CD3+TCR+ GFP/Luciferase human leukaemia in immunodeficientmice. (A) Serial bioluminescence imaging (BLI) of NSGmice (representative images from each cohort) following intraperitoneal administration of D-luciferin substrate showing elimination of CD3+ leukaemia by 3CAR T cells but not by untransduced T cells. (B) Kinetics of systemic leukaemia progression in mice where tumour burden following administration PBS (n=2), untransduced T cells (n=4) or 3CAR (n=5) effectors. Error bars represent median with interquartile range. Linear regression analysis showed significance between 3CAR vs untransduced (^{∗∗∗∗}P<0.0001) and 3CAR vs PBS (^{∗∗∗∗}P<0.0001) groups.(C) Average radiance values at termination (d18) indicated significant difference in disease burden between untransduced and 3CAR effector injected groups (^{∗}, P=0.0159 by Mann-Whitney U test). Error bars represent median with interquartile range.(D) Representative flow cytometry plots of T cells (gated on hCD45+CD2+GFP- expression) in bone marrow (BM). (E) Representative flow cytometry plots of leukaemic T cell (CD3+TCR+GFP+ Jurkat targets) after effector challenge. (F) Representative flow cytometry plots of CART cells population (gated on hCD45+CD2+GFP- expression). (G) Proportion of GFP+ Jurkat leukemia or GFP- effector T cells out of CD45+CD2+ population in bone marrow of untransduced or 3CAR injected mice. Red marking highlights an animal which exhibited selective outgrowth of GFP+CD3- Jurkats after 3CAR therapy.

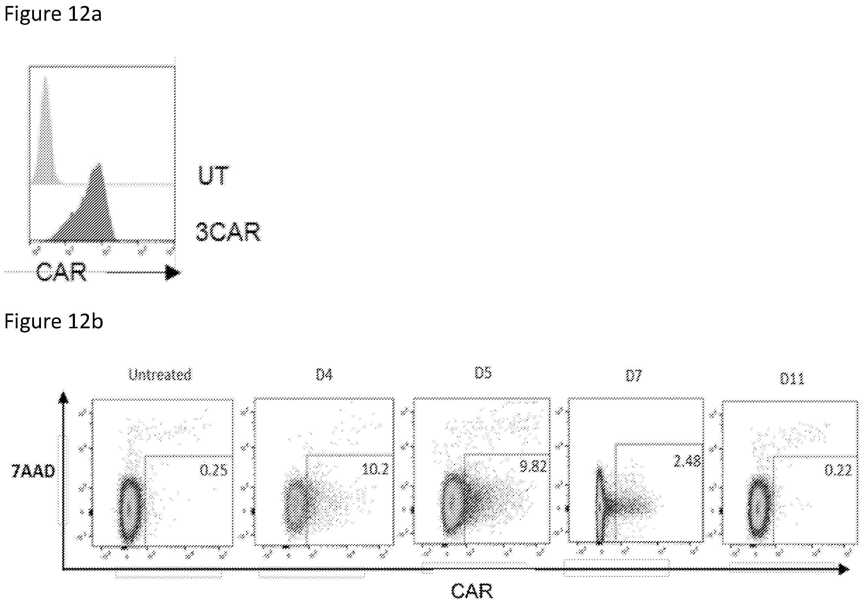

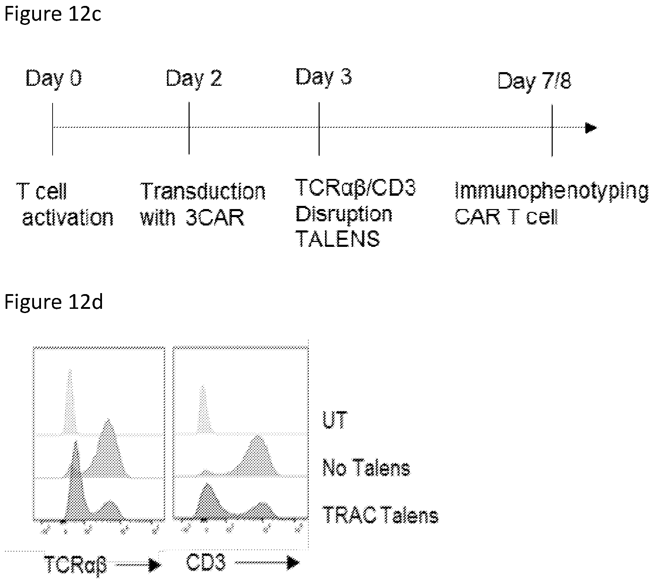

Figure 12: Generation of 3CAR T cells by conventional LV transduction followed by TRAC gene editing results in poor cell yields. A) Integrity of 3CAR cell surface expression verified on 293T cells transduced with LV-3CARand stained with F(ab). B) Representative flow cytometry plots of human T cells transduced with LV-3CAR showing transient CAR expressionC) T cells were activated with anti-CD3/CD28 reagent and 48 hrs later transduced with LV followed by electroporation with TRAC TALENS. D) On day 7 cells were phenotyped for CAR expression TCR and CD3 expression in cells transduced with LV-3CARand then treated with TRAC specific TALENs.

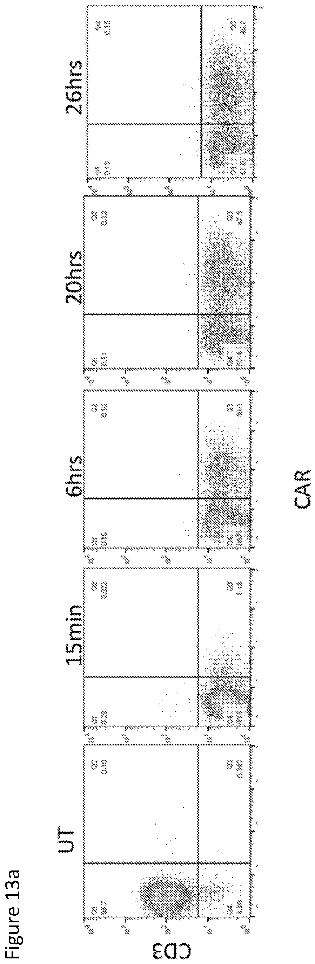

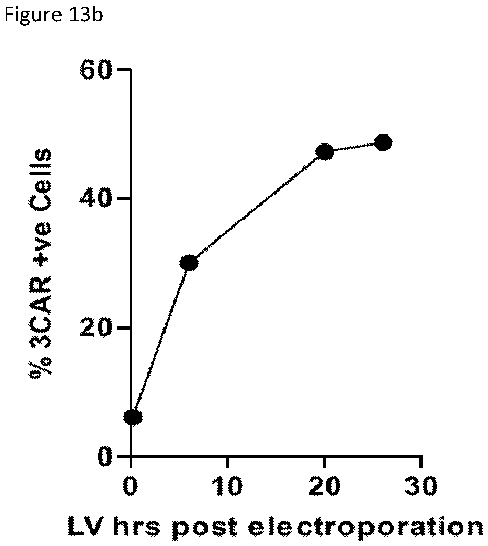

Figure 13: 3CAR T cell yields produced by TRAC ko prior to LV3CAR transduction. A) T cells were activated with anti-CD3/CD28 reagent and 48 hrs later electroporated with TRAC TALENS. Cells were then transduced with LV-3CAR at anMOI at various time points post electroporation. On day 7 cells were phenotyped for CAR CD3 expression. B)%o f 3CAR+ve cells from A.

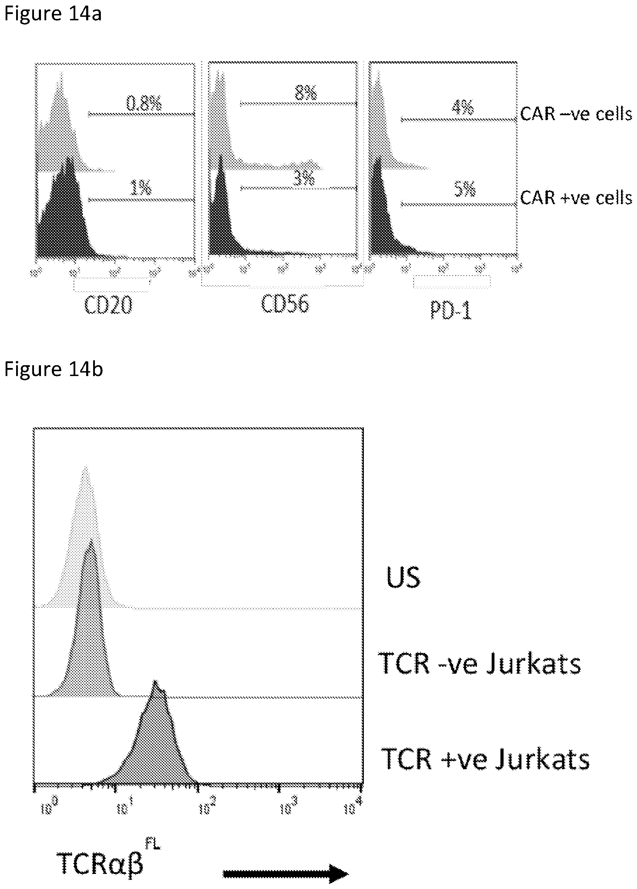

Figure 14: Following TALEN TRAC mRNA exposure, 2m T cells were transduced and expanded over 17 days. Cells were stained for Viability 7AAD, CAR, PD-1, CD20, and CD56 expression on d13. B) TCR+ve andTCR-ve Jurkat target cells were stained for TCR expression prior to coculture experiments.

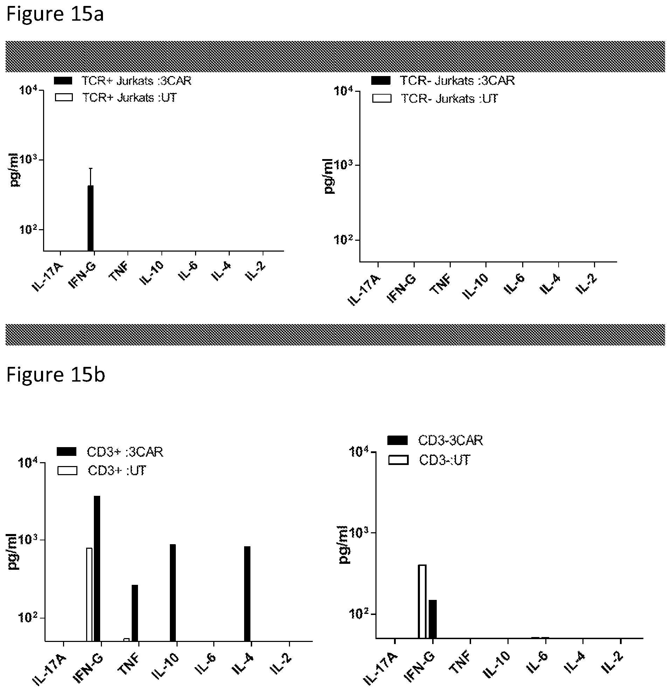

Figure 15: A) Cytokine production of 3CAR T cells when co-cultured with either GFP+ CD3/TCR+ or GPP+ CD3/TCR- Jurkats target cells asmeasured by CBA. B) Cytokine production of 3CAR T cells when co-cultured with either healthy donor CD3+, CD3- or PBMC target cells.

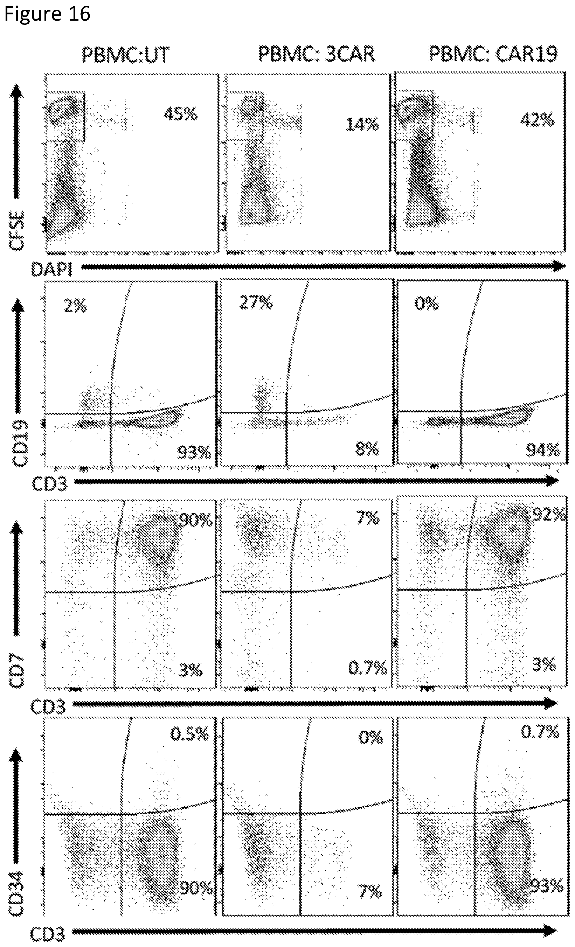

Figure 16: CAR, CAR19 or untransduced T cells were cocultured with CSFE loaded PBMC at the effector target ratio of 1:1 for 24hr. Top panel shows representative frequency of gated CSFE+ tumour cells at the end of coculture. Dot plots below show representative frequency of surface antigen markersCD3, CD19, CD7 and CD34 on gated CSFE+ tumour cells.

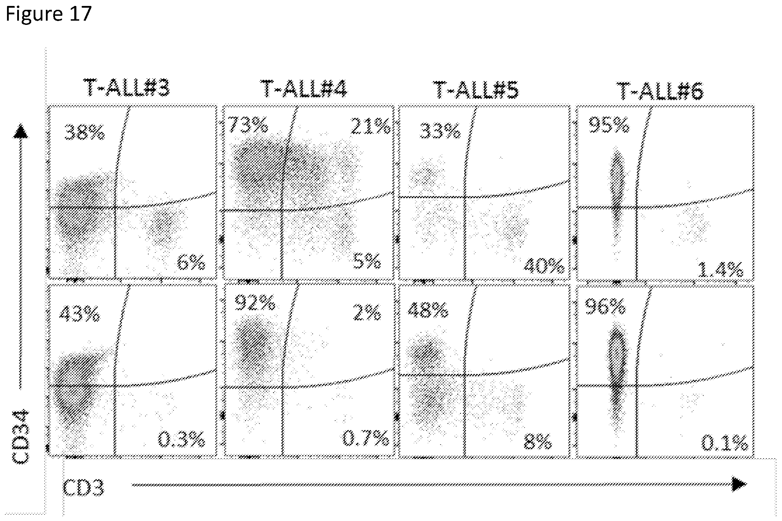

Figure 17: 3CAR (lower panels) or untransduced T cells (upper panels) were cocultured with CSFE loaded T-ALL patient donor at the effector target ratio of 1:1 for 24hr. Dot plots show the frequency of CD3 and CD34 cells gated on CSFE+ T-ALL tumour cells. 4 out of 6 patient samples were positive for CD34 expression.

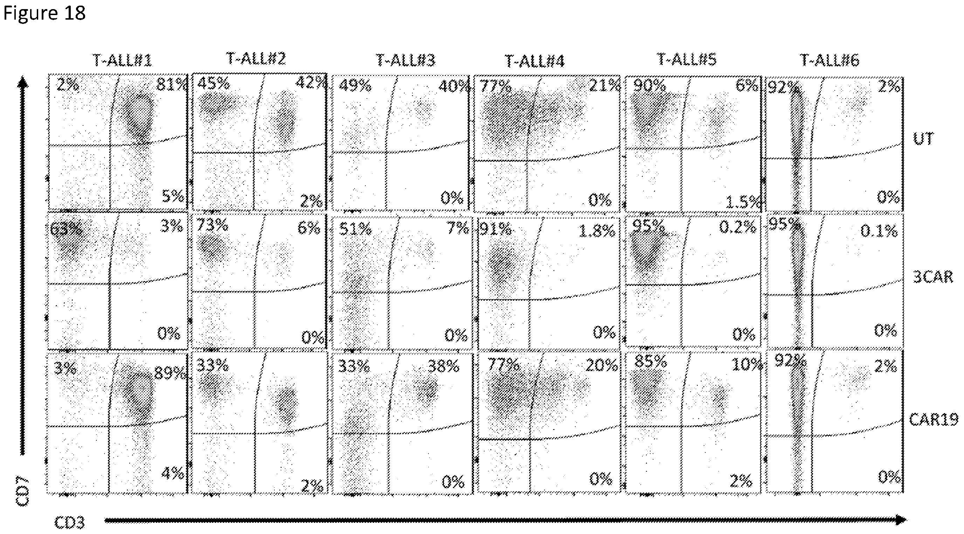

Figure 18: 3CAR, CAR19 or untransduced T cells were cocultured with CSFE loaded T-ALL patient donor at the effector target ratio of 1:1 for 24hr. Dot plots show the frequency of CD3 and CD7 cells gated on CSFE+ T-ALL tumour cells. All 6 patient samples are shown.

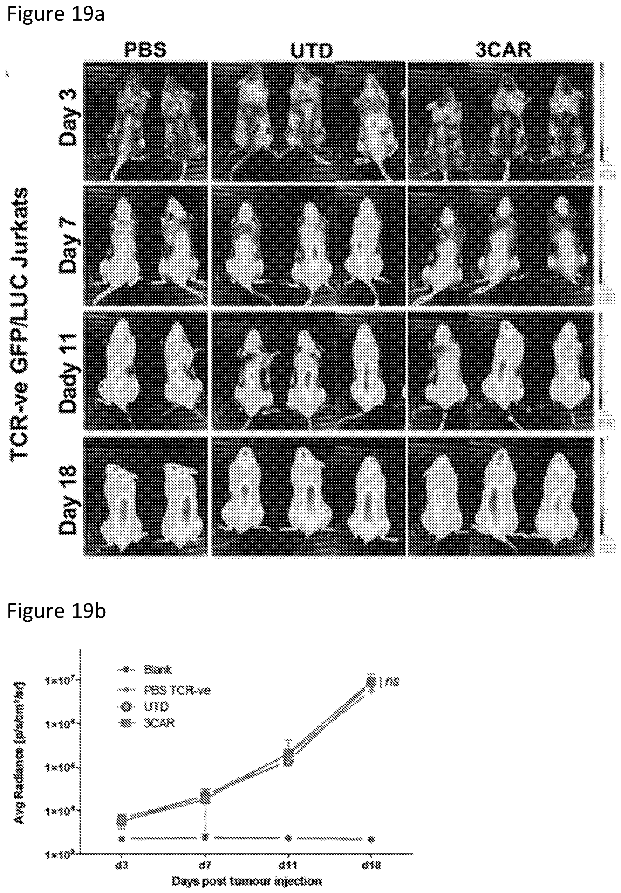

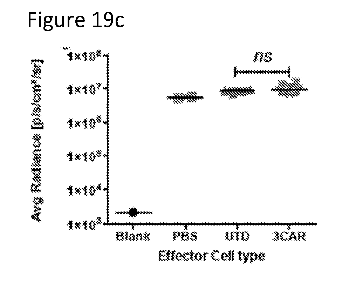

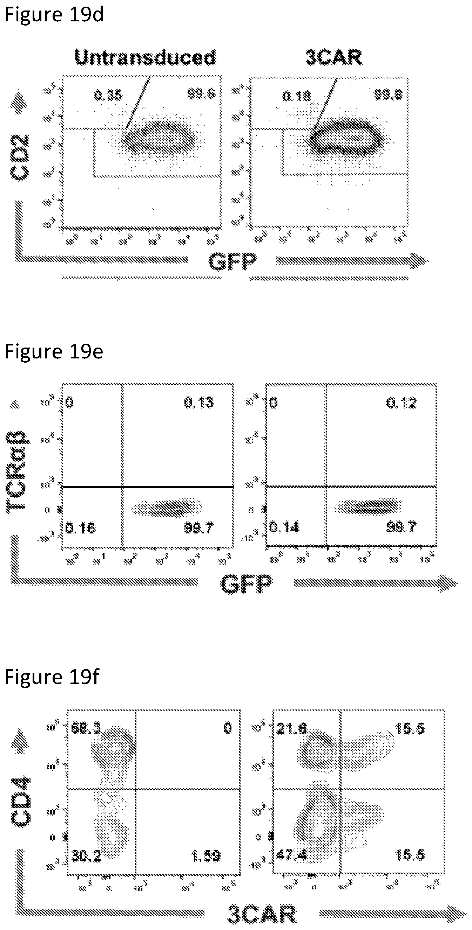

Figure 19: (A) Serial bioluminescence imaging (BLI) of NSGmice (representative images from each cohort). (B) Overall kinetics of systemic tumour progression inmice. Each line denotes the tumour burden following administration of effector groups, non-treated PBS injected (n=2), control untransduced T cell (n=4) and 3CAR (n=6) effectors. Error bars represent median with interquartile range. Linear regression analysis showed no significance between groups. (C) Average radiance values at termination (d18) indicate no significant difference in tumor burden between untransduced and 3CAR effector injected groups (P=0.7619 byMann-WhitneyU test). Error bars represent median with interquartile range. D) Representative flow cytometry plots of T cells population (gated on hCD45+CD2+GFP- expression) in bone marrow (BM). (E) Representative flow cytometry plots of leukaemic T cell (TCR- GFP+ Jurkat targets) after effector challenge. (F) Representative flow cytometry plots of CAR T cells polulation (gated on hCD45+CD2+GFP- expression).

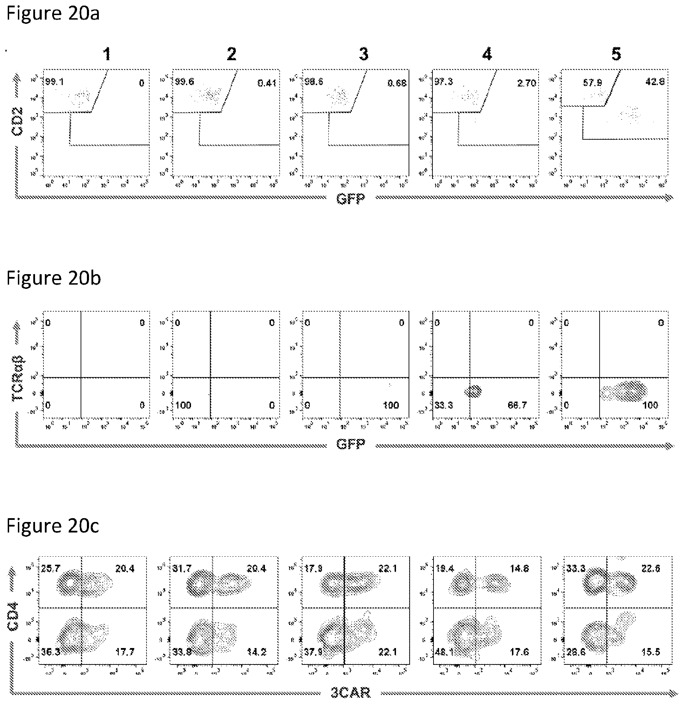

Figure 20: A) Flow cytometric analysis of 3CAR and tumour population in bone marrow of 5/5 immunodeficient NSG mice injected with TCR+eGFP+LUC+ Jurkat T cell tumour cells on day 0 and 3CAR effector cells on day 4. B) Bone marrow harvest from day 18 were gated on live,mCD11b- CD45+ cells and further phenotyped for CD2+ effector T cells or CD2+GFP+ Jurkat tumour cells. TCR+ tumour cells were further phenotyped for TCRαβ expression. Expansion of residual TCR- tumour cells due to impurity of starting population seen in animal 5. C) Effector cells stained for 3CARexpression show chimeric antigen receptor in 5/5 animals.

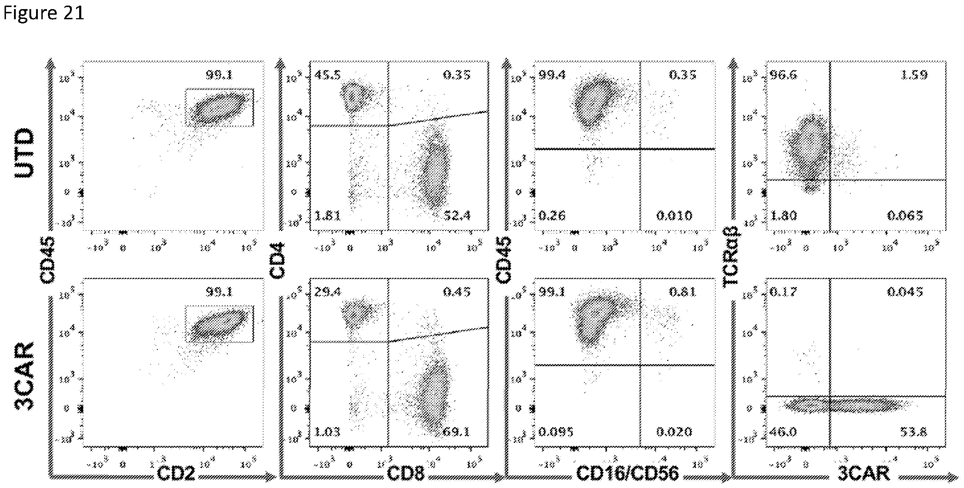

Figure 21: PBMCs were activated on day 0 and either treated with TALENs against TRAC or left untreated. TALEN treated cells were exposed to a single round of lentiviral transduction with 3CAR. Untreated cells were not transduced. Cells were expanded in culture for 16 days and phenotyped prior to cryopreservation for subsequent in vivo studies. Untreated/untransduced cells (UTD) and 3CAR cells were stained for CD45/CD2, CD4/CD8, CD16/CD56 and TCRαβ/3CAR.

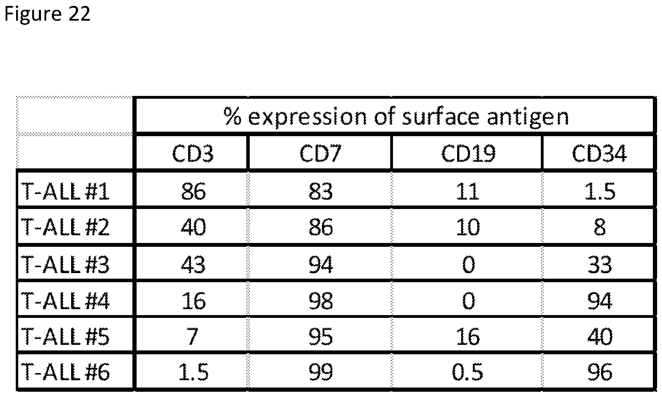

Figure 22: Table showing % expression of surface antigen.

### Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting.

In addition, as used in this specification and the appended claims, the singular forms "a", "an", and "the" include plural referents unless the content clearly dictates otherwise. Thus, for example, reference to "a CAR" includes "CARs", reference to "a T cell" includes two or more such T cells, reference to "a component" includes two or more such components, and the like.

### Generation of T-cell targeted T cells

The invention provides a method for producing a T cell expressing one or more chimeric antigen receptors (CARs) specific for CD3. The method comprises (i) eliminating endogenous expression of a T cell receptor (TCR)/CD3 complex component in a T cell, (ii) introducing a nucleic acid sequence encoding the one or more CARs to said T cell of (i), and subsequently (iii) expressing the one or more CARs of (ii) in said T cell of (i); wherein (i) is carried out 10 to 40 hours before (ii). Step (iii) is carried out after (i) and after (ii).

The method may comprise, after (iii), formulating the T cell for administration to an individual. For example, pharmaceutically compatible diluents or excipients may be added to the T cell. For instance, isotonic phosphate buffered saline, EDTA, DMSO and/or human albumin serum may be added to the T cell. Preferably, isotonic phosphate buffered saline, EDTA, DMSO and human albumin serum are added to the T cell to provide a formulation comprising the T cell, isotonic phosphate buffered saline, EDTA, DMSO and human albumin serum. The formulation may comprise about 7.5% DMSO, such as about 3%, about 4%, about 5%, about 6%, about 8%, about 9% or about 10% DMSO. The formulation may comprise about 4% human albumin serum, such as about 1%, about 2%, about 3%, about 5%, about 6% or about 7% human albumin serum.

The method may comprise, after (iii), cyropreserving the T cell. Cryopreservation may take place after the T cell is formulated for administration to an individual as set out above. Cells may be cryopreserved using a controlled rate freezer stored in the vapour phase of liquid nitrogen until required. If the method comprises cryopreserving the T cell, the T cell may be thawed before use, e.g. before administration to an individual. For example, the cell may be thawed at the bedside. The cell may be thawed in a waterbath. infused into a vein over a period of 5 minutes.

### T cells

The T cell may be a CD8+ T cell, or cytotoxic T cell. The T cell may be a CD4+ T cell, or helper T cell (T_{H} cell), such as a T_{H}1, TH2, TH3, TH17, TH9, or T_{FH} cells. The T cell may be a regulatory T cell (Treg). The T cell may be a naive, effector, memory, effector memory, central memory, memory stem T cell. The T cell may be a cord blood cell. The T cell may be a peripheral lymphocyte. The T cell may be expanded from PBMNCs. The T cell may be autologous with respect to an individual into which it is to be administered. The T cell may be allogeneic with respect to an individual into which it is to be administered. The T cell may be partially HLA-mismatched with respect to an individual into which it is to be administered.

Preferably, the T cell is generated from a cord blood T cell. The use of cord blood T cells is advantageous to the invention because they have a naive phenotype, an immense proliferative potential and potent *in vivo* activity in transplant recipients. Thus, the method of the invention may begin with a sample of cord blood. The sample of cord blood may be any type of sample. For instance, the sample of cord blood may be fresh cord blood or frozen cord blood. The sample of cord blood may have been derived from one individual. The sample of cord blood may have been derived from multiple individuals, i.e. a pooled cord blood sample.

Cord blood T cells may be obtained from the cord blood sample by separating cells that express CD62L from the sample. Any appropriate method may be used to separate cells that express CD62L from the sample. For instance, the cells that express CD62L may be separated from the sample based on their ability to bind an anti-CD62L antibody. Fluorescence activated cell sorting (FACS) or magnetic activated cell sorting (MACS) may be used to separate the cells that express CD62L from the sample. In MACS, magnetic beads are conjugated to the anti-CD62L antibody. Binding of the CD62L-expressing cells to the anti-CD62L antibody therefore tags the cells with magnetic beads. Magnetism can therefore be used to separate the tagged cells from the sample.

The separation step may be manually performed. Alternatively, the separation step may be performed in a system designed for the automated separation of cells. In one aspect, the system is configured for automated production of cord T cells. The system may be a CliniMacs system or a Miltenyi Prodigy system. Other automated cell separation systems are known in the art.

In some aspects, the T cell is stimulated before step (i) of the method of the invention. For instance, the T cell may be contacted with an anti-CD3 antibody and/or an anti-CD28 antibody. In this way, the T cell may be activated or expanded. The anti-CD3 antibody and/or the anti-CD28 antibody may be present on microbeads. Cytokines such as IL2, IL7, or IL15 may additionally be present alone or in combination.

### Elimination of endogenous expression of a TCR complex component

In step (i) of the method, endogenous expression of a TCR/CD3 complex component in a T cell is eliminated. Endogenous expression of CD5 and CD7 may be disrupted. For instance, endogenous expression of (a) a T cell receptor (TCR)/CD3 complex component may be eliminated and CD5 may be disrupted, (b) a T cell receptor (TCR)/CD3 complex component may be eliminated and CD7 may be disrupted, or (g) a T cell receptor (TCR)/CD3 complex component may be eliminated, and CD5 and CD7 may be disrupted in step (i) of the method. Endogenous expression of CD52, MHC and/or a molecule involved in a checkpoint pathway may also be disrupted.

Disruption of the expression of CD5 or CD7 refers to changing the amount of expression of CD5 or CD7 on the surface of the T-cell. Preferably, the expression of CD5 or CD7 is reduced or completely eliminated. A T-cell having reduced expression of CD5 or CD7 has a reduced amount of CD5 or CD7 on its surface. A T-cell with completely eliminated expression of the TCR/CD3 complex component, CD5 or CD7 has none of the TCR/CD3 complex component, CD5 or CD7 on its surface.

Methods for determining surface expression of TCR/CD3 complex components, CD5 or CD7 are known in the art. For instance, T cells surface-stained (i.e. stained without a permeablisation step) with an antibody or other molecule that binds to the TCR or CD3 elements, CD5 or CD7 may be analysed by flow cytometry or fluorescence microscopy. Using flow cytometry, reduction in the mean fluorescence intensity (MFI) of TCR/CD3 complex component, CD5 or CD7 surface-staining in study T cells compared to control T cells indicates a reduction in surface expression of the TCR/CD3 complex component, CD5 or CD7 respectively. For instance, the MFI (and thus surface expression) may be reduced by up to 100%, such as up to 99%, up to 98%, up to 95%, up to 90%, up to 85%, up to 80%, up to 75%, up to 70%, up to 60%, up to 50%, up to 40%, or up to 25%, in study T cells compared to control T cells. The absence of expression of a TCR/CD3 complex component, CD5 or CD7 is indicated by the absence of surface-staining for the TCR/CD3 complex component, CD5 or CD7 respectively (i.e. by an MFI equivalent or similar to that of a negative control sample).

The TCR/CD3 complex, otherwise known as the T-cell receptor complex, is a multimeric complex on the T-cell surface whose activation leads to the activation of the T-cell. The complex comprises (i) TCR, (ii) CD3 T-cell activation receptor. As set out below, the TCR comprises alpha (α) and beta (β) chains. The CD3 T-cell receptor comprises a CD3-gamma (CD3γ) chain, a CD3-delta (CD3δ) chain, two CD3-epsilon (CD3ε) chains and two zeta-chain (ζ-chain) accessory molecules.

TCRs allow for the antigen-specific activation of T-cells. Every T-cell expresses clonal TCRs which recognize specific peptide/MHC complex during physical contact between T-cell and antigen-presenting cell-APC (via MHC class II) or any other cell type (via MHC class I). The TCR is a disulfide-linked membrane-anchored heterodimeric protein normally consisting of the highly variable alpha (α) and beta (β) chains. Each chain of the TCR comprises two extracellular domains: a variable (V) region and a constant (C) region, both of immunoglobulin superfamily (IgSF) domain forming antiparallel β-sheets. The constant region is proximal to the cell membrane, followed by a transmembrane region and a short cytoplasmic tail, while the variable region binds to the peptide/MHC complex. The variable domain of the TCR α-chain and the TCR β-chain each have three hypervariable or complementarity determining regions (CDRs), that contribute to the TCR's specificity for a particular peptide/MHC complex. The variable region of the β-chain also has an additional area of hypervariability (HV4) that does not normally contact antigen. Subsets of T cells may express γδ TCR/CD3 complexes rather than αβ TCR and these cells can operate without MHC restriction

CD3 is required for the antigen-specific activation of T-cells. In particular, CD3 links antigen recognition by the TCR with intracellular signalling events downstream of the TCR CD3 zeta-chain. CD3 is a protein complex comprising six distinct chains. In mammals, CD3 comprises a CD3-gamma (CD3γ) chain, a CD3-delta (CD3δ) chain, two CD3-epsilon (CD3ε) chains and two zeta-chain (ζ-chain) accessory molecules. The CD3-gamma, CD3-delta and CD3-epsilon chains are highly related cell-surface proteins of the immunoglobulin superfamily comprising a single extracellular immunoglobulin domain. The transmembrane region of the CD3 chains is negatively charged, allowing the chains to associated with TCR chains, which are positively charged. The zeta-chain (also known as T-cell surface glycoprotein CD3 zeta-chain or CD247) plays an important role in coupling antigen recognition to several intracellular signal-transduction pathways. Low expression of the zeta-chain results in an impaired immune response.

Accordingly, TCR/CD3 normally comprises several components: TCR α-chain, TCR β-chain, CD3-gamma chain, CD3-delta chain, two CD3-epsilon chains, and two CD3- zeta-chains. In the method of the invention, endogenous expression of one or more of these components is eliminated. For example, endogenous expression of two or more, three or more, four or more, five or more, or six or more of these components may be eliminated. When endogenous expression of two or more of (i) TCR α-chain, (ii) TCR β-chain, (iii) CD3-gamma chain, (iv) CD3-delta chain, (v) CD3-epsilon chain, and (v) zeta-chain are eliminated, these TCR complex components may be eliminated in any combination, i.e. (i); (ii); (iii); (iv); (v); (i) and (ii); (i) and (iii); (i) and (iv); (i) and (v); (ii) and (iii); (ii and (iv); (ii) and (v); (iii) and (iv); (iii) and (v); (iv) and (v); (i), (ii) and (iii); (i), (ii) and (iv); (i), (ii) and (v); (i), (iii) and (iv); (i), (iii) and (v); (i), (iv) and (v); (ii), (iii) and (iv); (ii), (iii) and (v); (ii), (iv) and (v); (iii), (iv) and (v); (i), (ii), (iii) and (iv); (i), (ii), (iii) and (v); (i), (ii), (iv) and (v); (i), (iii), (iv) and (v); (ii), (iii), (iv) and (v); or (i), (ii), (iii), (iv) and (v).

Endogenous expression of CD52 may be disrupted. CD52 is a glycoprotein present on the surface of mature lymphocytes, but not on the stem cells from which these lymphocytes were derived.

Endogenous expression of MHC may be disrupted. MHC class 1 may be disrupted by targeting the transporter associated with antigen processing (TAP1 or TAP2) locus, whichever method of disruption is used. MHC class 1 may be disrupted by Beta-2 microglobulin (B2M) locus, whichever method of disruption is used (see gene editing techniques below). MHC molecules may also be disrupted by targeting transcription factors controlling MHC expression such as CIITA, RFX5, RFXAP or RFXANK

Disrupting expression of MHC may reduce or eliminate the effects of using HLA-mismatched (or partially mismatched) allogeneic T cells in the method. Specifically, if allogeneic cells are used, allogeneic 3CAR T cells will be generated. In this case, consideration must be given to the consequences of potential HLA-mismatch between the donor and recipient. For all types of therapeutic cells, minimising human leukocyte antigen (HLA)-mismatch reduces rejection of the therapeutic cells by the patient, improving their longevity and therapeutic potential. Furthermore, it is particularly important to reduce HLA-mismatch for therapeutic immune cells or hematopoietic stem cells, due to their ability to cause graft versus host disease (GVHD) when transplanted to an HLA-mismatched patient. GVHD arises when the native T-cell receptor (TCR) of T cells in or arising from the donated tissue (the "graft") recognise antigens in the recipient (the "host") as foreign. Thus, transplanted T cells attack host cells and tissues, causing damage to the host organs.

Endogenous expression of a molecule involved in a checkpoint pathway, such as PD-1, may be disrupted. Disruption of checkpoint inhibitor pathways unleashes an immune system attack on cancer cell, such as malignant T cells.

### Gene editing

A gene editing technique may be used to eliminate endogenous expression of a TCR complex component, or disrupt endogenous expression of CD5, CD7, CD52, MHC and/or a molecule involved in a checkpoint pathway. Gene editing is a type of genetic engineering in which DNA is inserted, deleted or replaced in the genome of a living organism using engineered nucleases, or "molecular scissors." These nucleases create site-specific double-strand breaks (DSBs) at desired locations in the genome. The induced double-strand breaks are repaired through nonhomologous end-joining (NHEJ) or homologous recombination (HR), resulting in targeted mutations ("edits").

Four families of engineered nucleases may be used for gene editing: (a) meganucleases, (b) zinc finger nucleases (ZFNs), (c) transcription activator-like effector-based nucleases (TALENs), (d) mega-TALENs and (e) the CRISPR-Cas system. Techniques using each of these engineered nucleases are well known in the art. All of these genome editing methods can disrupt a gene, entirely knocking out all of its output. One or more of these techniques may be used alone or in any combination to eliminate endogenous expression of a TCR complex component in the method of the invention, i.e. (a); (b); (c); (d); (e); (a) and (b); (a) and (c); (a) and (d); (a) and (e); (b) and (c); (b) and (d); (b) and (e); (c) and (d); (c) and (e); (d) and (e); (a), (b) and (c); (a), (b) and (d); (a), (b) and (e); (a), (c) and (d); (a), (c) and (e); (a), (d) and (e); (b), (c) and (d); (b), (c) and (e); (b), (d) and (e); (c), (d) and (e); (a), (b), (c) and (d); (a), (b), (c) and (e); (a), (b), (d) and (e); (a), (c), (d) and (e); (b), (c), (d) and (e); and (a), (b), (c), (d) and (e).

When the gene editing technique is used to eliminate expression of a TCR complex component (and, optionally, to disrupt expression of CD5 and/or CD7), the gene editing technique may target one or more of (i) the TRAC locus; (ii) a locus controlling TCR beta expression; (iii) a locus controlling TCR gamma expression; (iv) a locus controlling TCR delta expression (v) a locus controlling expression of a CD3 chain. For instance, the gene editing technique may target (i); (ii); (iii); (iv); (v); (i) and (ii); (i) and (iii); (i and (iv); (i) and (v); (ii) and (iii); (ii and (iv); (ii) and (v); (iii) and (iv); (iii) and (v); (iv) and (v); (i), (ii) and (iii); (i), (ii) and (iv); (i), (ii) and (v); (i), (iii) and (iv); (i), (iii) and (v); (i), (iv) and (v); (ii), (iii) and (iv); (ii), (iii) and (v); (ii), (iv) and (v); (iii), (iv) and (v); (i), (ii), (iii) and (iv); (i), (ii), (iii) and (v); (i), (ii), (iv) and (v); (i), (iii), (iv) and (v); (ii), (iii), (iv) and (v); or (i), (ii), (iii), (iv) and (v). CD3 chains are described above. The CD3 chain may be CD3y, CD3δ, CD3ε or CD3ζ.

### CD3-specific CAR (3CAR)

As set out in Example 2 and shown in Figure 2, expression of 3CAR in T cells expressing their TCR/CD3 complex results in poor cell yields because CD3e expressed on the cells is recognised by the 3CAR of other T cells, triggering cytotoxic effects. For other CAR-expressing T cells, expression of the TCR/CD3 complex is disrupted after expression of other CARs (such as CARs against CD19) is introduced. This TCR/CD3 complex disruption is known in the art to support the production of universal, CAR T cells with reduced allo-reactivity. However, a similar approach using 3CAR has not been successful because endogenous CD3e is recognised by the 3CAR as soon as the protein is expressed (Example 2, Figure 3).

The present inventors have surprisingly found that by eliminating expression of a TCR/CD3 complex component before a 3CAR is introduced to the T cells is expressed, suitable yields of engineered, CD3-specific T cells can be generated. Accordingly, in the present invention, endogenous expression of a TCR complex component in a T cell is eliminated. Subsequently, a 3CAR is expressed. The 3CAR is encoded by a nucleic acid sequence which is introduced to the T cell after the endogenous expression of a TCR/CD3 complex component is eliminated. The CD3-specific T cells may additionally have disrupted expression of endogenous CD5 and/or CD7.

Thus, one or more nucleic acid sequences encoding a CD3-specific CAR is introduced to the T cell after steps are taken to eliminate endogenous expression of a TCR/CD3 complex component. The CAR protein is expressed after endogenous expression of the TCR/CD3 complex component is eliminated. The nucleic acid encoding the CAR is introduced to the T cell 10 to 40, such as 15 to 35 or 20 to 30, hours after endogenous expression of a TCR complex component is eliminated. In other words, step (i) of the method of the invention is carried out 10 to 40, such as 15 to 35 or 20 to 30, hours before step (ii). This particular window between step (i) and step (ii) improves the efficiency of generating T-cell targeted T cells, as shown in the Examples. This is surprising, given that Osborn et al. (2016) Molecular Therapy, Volume 25, No.3, pages 570 to 581) demonstrated that disrupting the expression of the T cell receptor before introducing a CAR is detrimental to T cell viability. Increasing the window between step (i) and step (ii) to e.g. 20 to 30 hours would not, therefore, have been predicted to improve the efficiency of generation.

The method may be used to product a T cell expressing one or more CARs specific for CD5 and/or CD7. Thus, step (ii) of the method may comprise the introduction of one or more of a nucleic acid sequence encoding a nucleic acid sequence encoding a CD5-specific CAR, and a nucleic acid sequence encoding a CD7-specific CAR. For example, step (ii) may comprise introducing a nucleic acid sequence encoding a CD3-specific CAR to said T cell of (i). Step (ii) may comprise introducing a nucleic acid sequence encoding a CD3-specific CAR and a nucleic acid sequence encoding a CD5-specific CAR to said T cell of (i). Step (ii) may comprise introducing a nucleic acid sequence encoding a CD3-specific CAR and a nucleic acid sequence encoding a CD7-specific CAR to said T cell of (i). Step (ii) may comprise introducing a nucleic acid sequence encoding a CD3-specific CAR, a nucleic acid sequence encoding a CD5-specific CAR and a nucleic acid sequence encoding a CD7-specific CAR to said T cell of (i). In any of these cases, step (iii) comprises expressing the CAR or CARs of (ii) in the T cell of (i).

When two or more CAR-encoding nucleic acid sequences are introduced in step (ii), each CAR-encoding nucleic acid sequence may be introduced using a separate vector. Alternatively, each CAR-encoding nucleic acid sequence may be introduced using the same vector. When two CAR-encoding nucleic acid sequences are introduced in step (ii), the CAR-encoding nucleic acid sequences may be encoded by a nucleic acid sequence encoding a bispecific CAR

The CAR protein may be expressed immediately after the nucleic acid sequence encoding the CAR is introduced to the T cell. Alternatively, there may be a delay in expression of the CAR protein following introduction of the nucleic acid sequence encoding the CAR to the T cell. The delay may be around 24 hours in duration. The delay may be 12, 13, 14, 15, 16, 17, 18, 19, 20, 21, 22, 23, 24, 25, 26, 27, 28, 29, 30, 31, 32, 33, 34, 35 or 36 in duration. The delay may be 12 to 36 hours in duration, such as 12 to 34, 12 to 32, 12 to 30, 12 to 28, 12 to 26, 12 to 24, 12 to 22, 12 to 20, 12 to 18, 12 to 16, 12 to 14, 14 to 36, 14 to 34, 14 to 32, 14 to 30, 14 to 28, 14 to 26, 14 to 24, 14 to 22, 14 to 20, 14 to 18, 14 to 16, 16 to 36, 16 to 34, 16 to 32, 16 to 30, 16 to 28, 16 to 26, 16 to 24, 16 to 22, 16 to 20, 16 to 18, 18 to 36, 18 to 34, 18 to 32, 18 to 30, 18 to 28, 18 to 26, 18 to 24, 18 to 22, 18 to 20, 20 to 36, 20 to 34, 20 to 32, 20 to 30, 20 to 28, 20 to 26, 20 to 24, 20 to 22, 22 to 36, 22 to 34, 22 to 32, 22 to 30, 22 to 28, 22 to 26, 22 to 24, 24 to 36, 24 to 34, 24 to 32, 24 to 30, 24 to 28, 24 to 26, 26 to 36, 26 to 34, 26 to 32, 26 to 30, 26 to 28, 28 to 36, 28 to 34, 28 to 32, 28 to 30, 30 to 36, 30 to 34, 30 to 32, 32 to 36, 32 to 34 or 34 to 36 hours. The delay may occur because the vector comprising the nucleic acid sequence encoding the CAR has to enter cells, reverse transcribe, translocate to nucleus, integrate and then express mRNA and then protein. A further period of delay may occur when TALENs are used as the gene editing technique and the cells are cultured at 30°C to 32°C for a period of time. The duration of the further period of delay will depend on the length of culture at this temperature. For instance, the cells may be cultured for up to 12 hours, 24 hours, 36 hours, 48 hours or 72 hours, in which case the duration of the further delay up to12 hours, 24 hours, 36 hours, 48 hours or 72 hours respectively.

Expression of the CAR protein may be apparent from the presence of CAR protein on the surface of the T cell. Methods for identifying the presence of CAR protein on the surface of the T cell are well known in the art. For instance, flow cytometry may be used. Expression of the CAR protein may be apparent from its functional effects on the T cell in which it is expressed. In particular, the T cell is antigen-specific for CD3 when the 3CAR protein is expressed. The T cell is antigen-specific for CD5 when the 5CAR protein is expressed. The T cell is antigen-specific for CD7 when the 7CAR protein is expressed. Methods for determining the CD3-specificify, CD5-specificify or CD7-specificify of the T cell are known in the art. For instance, a CD3-specific T cell may be capable of targeting or killing a CD3-expressing cell (such as a non-engineered T cell) in an *in vitro* assay.

The CAR may be expressed by the T cell about one, two, three, four, five, six or seven days after elimination of endogenous expression of a TCR complex component. For example, the CAR may be expressed by the T cell about 0 hours, about 6 hours, about 12 hours, about 24 hours, about 36 hours or about 48 hours after elimination of endogenous expression of a TCR/CD3 complex component. The CAR may be expressed by the T cell about 6 to 24 hours after elimination of endogenous expression of a TCR complex component. For instance, the CAR may be expressed by the T cell 6 to 8, 6 to 10, 6 to 12, 6 to 14, 6 to 16, 6 to 18, 6 to 20, 6 to 22, 6 to 24, 8 to 10, 8 to 12, 8 to 14, 8 to 16, 8 to 18, 8 to 20, 8 to 22, 8 to 24, 10 to 12, 10 to 14, 10 to 16, 10 to 18, 10 to 20, 10 to 22, 10 to 24, 12 to 14, 12 to 16, 12 to 18, 12 to 20, 12 to 22, 12 to 24, 14 to 16, 14 to 18, 14 to 20, 14 to 22, 14 to 24, 16 to 18, 16 to 20, 16 to 22, 16 to 24, 18 to 20, 18 to 22, 18 to 24, 20 to 22, 20 to 24 or 22 to 24 hours after elimination of endogenous expression of a TCR complex component. The CAR may be expressed by the T cell 6, 7, 8, 9, 10, 11, 12, 13, 14, 15, 16, 17, 18, 19, 20, 21, 22, 23, 25, 26, 27, 28, 29, 30, 31, 32, 33, 34, 35, 37, 38, 39, 40, 41, 42, 43, 44, 45, 46, or 47 hours after elimination of endogenous expression of a TCR complex component.

The T cell may express 3CAR on its surface and/or be antigen specific for CD3 respectively about one, two, three, four, five, six or seven days after elimination of endogenous expression of a TCR complex component. For example, the T cell may express 3CAR on its surface and/or be antigen specific for CD3 about 0 hours, about 6 hours, about 12 hours, about 24 hours, about 36 hours or about 48 hours after elimination of endogenous expression of a TCR/CD3 complex component. The T cell may express 3CAR on its surface and/or be antigen specific for CD3 about 6 to 24 hours after elimination of endogenous expression of a TCR complex component. For instance, the T cell may express 3CAR on its surface and/or be antigen specific for CD3 6 to 8, 6 to 10, 6 to 12, 6 to 14, 6 to 16, 6 to 18, 6 to 20, 6 to 22, 6 to 24, 8 to 10, 8 to 12, 8 to 14, 8 to 16, 8 to 18, 8 to 20, 8 to 22, 8 to 24, 10 to 12, 10 to 14, 10 to 16, 10 to 18, 10 to 20, 10 to 22, 10 to 24, 12 to 14, 12 to 16, 12 to 18, 12 to 20, 12 to 22, 12 to 24, 14 to 16, 14 to 18, 14 to 20, 14 to 22, 14 to 24, 16 to 18, 16 to 20, 16 to 22, 16 to 24, 18 to 20, 18 to 22, 18 to 24, 20 to 22, 20 to 24 or 22 to 24 hours after elimination of endogenous expression of a TCR complex component. The T cell may express 3CAR on its surface and/or be antigen specific for CD3 6, 7, 8, 9, 10, 11, 12, 13, 14, 15, 16, 17, 18, 19, 20, 21, 22, 23, 25, 26, 27, 28, 29, 30, 31, 32, 33, 34, 35, 37, 38, 39, 40, 41, 42, 43, 44, 45, 46, or 47 hours after elimination of endogenous expression of a TCR complex component.

The T cell may be cultured or incubated between elimination of endogenous expression of a TCR complex component and introduction and/or expression of a nucleic acid sequence encoding a CD3-specific CAR. For instance, the T cell may be cultured at 30°C to 37°C, such as at 31°C, 32°C, 33°C, 34°C, 35°C or 36°C. Standard culture conditions are well known in the art.

The CD3-specific CAR (3CAR) may comprise a scFV from a CD3-specific antibody. The CD3-specific antibody may be a monoclonal antibody. The CD3-specific antibody may be a human antibody. The CD3-specific antibody may be a humanised antibody. The CD3-specific antibody may be a non-human antibody, such a, canine, equine, bovine, ovine, porcine, murine, feline, leporine, cavine or camelid antibody. If the CD3-specific antibody is a non-human antibody, it may comprise one or more human constant domains. For instance, the CD3-specific CAR may comprise a scFV from any of the following CD3-antibodies, which are well known in the art: OKT3, RIV-9, UCHT1, SK7, MEM57, MEM92, or HIT3A. The CD3-specific CAR may comprise a scFV from the OKT3 antibody. The CD3-specific CAR may comprise a scFV from an antibody that competes for CD3 binding with one or more of OKT3, RIV-9, UCHT1, SK7, MEM57, MEM92, or HIT3A. The CD3-specific CAR may comprise a scFV from an antibody that competes for CD3 binding with the OKT3 antibody.

The CD5-specific CAR (5CAR) may comprise a scFV from a CD5-specific antibody. The CD5-specific antibody may be a monoclonal antibody. The CD5-specific antibody may be a human antibody. The CD5-specific antibody may be a humanised antibody. The CD5-specific antibody may be a non-human antibody, such a, canine, equine, bovine, ovine, porcine, murine, feline, leporine, cavine or camelid antibody. If the CD5-specific antibody is a non-human antibody, it may comprise one or more human constant domains.

The CD7-specific CAR (7CAR) may comprise a scFV from a CD7-specific antibody. The CD7-specific antibody may be a monoclonal antibody. The CD7-specific antibody may be a human antibody. The CD7-specific antibody may be a humanised antibody. The CD7-specific antibody may be a non-human antibody, such as a canine, equine, bovine, ovine, porcine, murine, feline, leporine, cavine or camelid antibody. If the CD7-specific antibody is a non-human antibody, it may comprise one or more human constant domains.

The CAR may comprise a cytoplasmic domain comprising an activation domain. The activation domain serves to activate the T cell following engagement of the extracellular domain (e.g. scFv). For instance, the cytoplasmic domain may comprise one or more of a 41BB activation domain, a CD3ζ activation domain and a CD3e activation domain. Preferably, the cytoplasmic domain comprises a 41BB activation domain and/or a CD3ζ activation domain.

The CAR may comprise a transmembrane domain. The transmembrane domain serves to transmit activation signals to the cytoplasmic signal transduction domains following ligand binding of the extracellular domains (e.g. scFv). The CAR may comprise a spacer. The spacer connects the transmembrane domain to the extracellular domain. The spacer may confer steric effects that influence the strength of activation, cytotoxicity and signaling from the target cell and its surface receptors.

The nucleic acid sequence encoding the CAR may be introduced to the T cell using any method known in the art. In particular, the T cell may be transfected or transduced with the nucleic acid sequence. The CAR may be introduced to the T cell using a vector.

The term "transduction" may be used to describe virus mediated nucleic acid transfer. A viral vector may be used to transduce the cell with the one or more constructs. Conventional viral based expression systems could include retroviral, alpha-retroviral, lentivirus, adenoviral, adeno-associated (AAV) and herpes simplex virus (HSV) vectors for gene transfer. Non-viral transduction vectors include transposon based systems including PiggyBac and Sleeping Beauty systems.. Methods for producing and purifying such vectors are know in the art. The vector is preferably a vector of the invention. Cord blood T cells may be transduced using any method known in the art. Transduction may be in vitro or ex vivo.

The term "transfection" may be used to describe non-virus-mediated nucleic acid transfer. The cord blood T cells may be transfected using any method known in the art. Transfection may be in vitro or ex vivo. Any vector capable of transfecting the cord blood T cells may be used, such as conventional plasmid DNA or RNA transfection. A human artificial chromosome and/or naked RNA may be used to transfect the cell with the nucleic acid sequence or nucleic acid construct. Human artificial chromosomes are described in e.g. Kazuki et al., Mol. Ther. 19(9): 1591-1601 (2011), and Kouprina et al., Expert Opinion on Drug Delivery 11(4): 517-535 (2014). Alternative non-viral delivery systems include DNA plasmids, naked nucleic acid, and nucleic acid complexed with a delivery vehicle, such as a liposome. Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam^{™} and Lipofectin^{™}). Cationic and neutral lipids that are suitable for efficient receptor-recognition lipofection of polynucleotides include those of Feigner, WO 91/17424; WO 91/16024. The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

Nanoparticle delivery systems may be used to transfect the T cell with the nucleic acid sequence. Such delivery systems include, but are not limited to, lipid-based systems, liposomes, micelles, microvesicles and exosomes. With regard to nanoparticles that can deliver RNA, see, e.g., Alabi et al., Proc Natl Acad Sci U S A. 2013 Aug 6;110(32):12881-6; Zhang et al., Adv Mater. 2013 Sep 6;25(33):4641-5; Jiang et al., Nano Lett. 2013 Mar 13; 13(3): 1059-64; Karagiannis et al., ACS Nano. 2012 Oct 23;6(10):8484-7; Whitehead et al., ACS Nano. 2012 Aug 28;6(8):6922-9 and Lee et al., Nat Nanotechnol. 2012 Jun 3;7(6):389-93. Lipid Nanoparticles, Spherical Nucleic Acid (SNA^{™}) constructs, nanoplexes and other nanoparticles (particularly gold nanoparticles) are also contemplated as a means for delivery of a construct or vector in accordance with the invention.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectAmine, fugene and transfectam.

The T cell may be transfected or transduced under suitable conditions. Suitable conditions are known in the art. For instance, the T cell may be transfected or transduced following activation with combinations of antibodies such as anti-CD3 and anti-CD28 which may be conjugated to beads or polymers and used with or without cytokines such as IL2, IL7, and IL15, used alone or in combination. The T cell and agent or vector may, for example, be contacted for between five minutes and ten days, preferably from an hour to five days, more preferably from five hours to two days and even more preferably from twelve hours to one day after activation.

The nucleic acid sequence transduced or transfected into the T cell gives rise to expression of 3CARin the T cells. Methods for determining expression of CARs are known in the art. For instance, flow cytometry for FAB surface staining may be used to detect CAR expression.

If the nucleic acid sequence is transduced into the T cell, the vector used for transduction may comprise a further nucleic acid sequence encoding another molecule useful to the generation of 3CARcells, such as a suicide gene. Suicide genes are discussed in more detail below.

### Suicide gene

The method of the invention may comprise (ii) introducing a nucleic acid sequence encoding a suicide gene to the T cell.

Suicide genes are well known in the art. The suicide gene may be herpes simplex virus thymidine kinase, or an inducible caspase. The suicide gene may encode an epitope recognised by a monoclonal antibody specific for HerB2, EGFR or CD20.

The suicide gene provides an "off switch" that allows the 3CAR T cells produced by the method to be eliminated from an individual into whom they are administered. This provides a safety mechanism to stop the effects of the 3CAR T cells when their advantageous (therapeutic) effects are outweighed by their deleterious effects.

The nucleic acid sequence encoding the suicide gene may be introduced to the T cell together with the nucleic acid sequence encoding the 3CARor at the same time as the nucleic acid sequence encoding the 3CAR. The nucleic acid sequence encoding the suicide gene may be introduced to the cell using any method known in the art. The nucleic acid sequence encoding the suicide gene may be transfected or transduced into the T cell. Transfection and transduction are known in the art and are set out in detail above. The nucleic acid sequence encoding the suicide gene may be introduced to the cell using a vector comprising the nucleic acid sequence encoding the suicide gene. Suitable vectors are described above. The vector may also comprise a nucleic acid sequence encoding a 3CAR

Accordingly, the nucleic acid sequence encoding the 3CAR and the nucleic acid sequence encoding the suicide gene may be introduced to the T cell using the same vector. The nucleic sequence encoding the suicide gene may be linked to the nucleic acid sequence encoding the CD3-specific CAR R by an IRES element or a nucleic acid sequence encoding a self-cleaving peptide. The nucleic sequence encoding the suicide gene may linked to the nucleic acid sequence encoding the CD3-specific CAR such that the suicide gene and the CAR are expressed as an in-frame fusion construct.

### Multiple knockdown and CAR expression

Step (i) of the method of the invention comprises eliminating endogenous expression of a T cell receptor (TCR)/CD3 complex component in a T cell. Step (i) may, for example, comprise disrupting endogenous expression of (a) a T cell receptor (TCR)/CD3 complex component and CD5, (b) a T cell receptor (TCR)/CD3 complex component and CD7, or (c) a T cell receptor (TCR)/CD3 complex component, CD5 and CD7.

Step (ii) of the method comprises introducing a nucleic acid sequence encoding the one or more CARs specific got CD3 to said T cell of (i). Step (ii) may, for example, comprise introducing (t) a nucleic acid sequence encoding a CD3-specific CAR and a nucleic acid sequence encoding a CD5-specific CAR, (u) a nucleic acid sequence encoding a CD3-specific CAR and a nucleic acid sequence encoding a CD7-specific CAR, or (v) a nucleic acid sequence encoding a CD3-specific CAR, a nucleic acid sequence encoding a CD5-specific CAR and a nucleic acid sequence encoding a CD7-specific CAR to said T cell of (i).

Step (i) of the method comprises disrupting endogenous expression of the target corresponding to the specificity of the CD3-specific CAR that is introduced in step (ii). The endogenous expression of other targets may also be disrupted. In other words, CD3-specific CAR is to be introduced in step (ii), and so step (i) comprises eliminating endogenous expression of a TCR/CD3 complex component. Endogenous expression of CD5 and/or CD7 may optionally be disrupted. Preferably, the options set out for steps (i) and (ii) are combined as follows (t) and (a); (t) and (c); (u) and (b); (u) and (c); or (v) and (c).

### 3CART cells

The disclosure provides, for illustrative purposes, a T cell expressing one or more CARs specific for CD3, and lacking expression an endogenous TCR complex component (a 3CAR T cell), wherein the T cell is obtained using the method of the invention.

The T cell may have disrupted endogenous expression of (a) a T cell receptor (TCR) complex component, (b) a T cell receptor (TCR) complex component and CD5, (c) a T cell receptor (TCR) complex component and CD7, or (d) a T cell receptor (TCR) complex component, CD5 and CD7. The T cell may express (t) a CD3-specific CAR, (u) a CD3-specific CAR and a CD5-specific CAR, (v) a CD3-specific CAR and a CD7-specific CAR, or (w) a CD3-specific CAR, a CD5-specific CAR and a CD7-specific CAR.

The T cell may have disrupted endogenous expression of the target corresponding to the specificity of a CAR that it expresses. The T cell may also have distruped endogenous expression of other targets. That is, the T cell expresses a CD3-specific CAR and lacks endogenous expression of a TCR complex component. The T cell may optionally have disrupted expression of CD5 and/or CD7. Possible combinations of the recited CAR expressions and disruptions include e.g: (t) and (a); (t) and (b); (t) and (c); (t) and (d); (u) and (b); (u) and (d); (v) and (c); (v) and (d); (or (w) and (d).

In any case, the T cells may express a suicide gene. CD3-specific, CD5-specific and CD7-specific CARS, TCR complex components and suicide genes are described in detail above.

The T cell targets T cells. Therefore, the T cell may be used to target malignant T cells, or unwanted T cells, for example to treat cancer or ablate host immunity.

The 3CAR T cell has disrupted expression of an endogenous TCR complex component. Thus, the 3CAR T cell has a reduced ability to be recognised and /or attacked by T cells expressing an antigen receptor (such as a T cell receptor or a CAR) specific for an endogenous TCR complex component. Therefore, the 3CAR T cell has improved longevity, because it is not recognised and attacked by other T cell-targeted T cells. The 3CAR T cell-targeted T cell has a reduced susceptibility to fratricide.

The T cell may be any type of T cell. The T cell may be a CD4+ T-cell, or helper T-cell (T_{H} cell), such as a T_{H}1, TH2, TH3, TH17, TH9, or T_{FH} cell. The T cell may be a regulatory T-cell (Treg). The T cell is preferably a CD8+ T-cell, or cytotoxic T-cell. The T cell may be a naive, effector, memory, effector memory, central memory, memory stem T cell. The T cell may be a cord blood cell. The T cell may be a peripheral lymphocyte. The T cell may be expanded from PBMNCs. The T cell may be autologous with respect to an individual into which it is to be administered. The T cell may be allogeneic with respect to an individual into which it is to be administered. The T cell may be partially HLA-mismatched with respect to an individual into which it is to be administered.

A T cell expressing a CD3-specific CAR (3CAR) may comprise one or more, such as two or more, three or more, four or more, five or more or ten or more, nucleic acid sequences encoding a 3CAR. When the 3CAR T cell comprises two or more sequences encoding a 3CAR, the sequences may encode the same 3CAR or different 3CARs.

A T cell expressing a CD5-specific CAR (5CAR) may comprise one or more, such as two or more, three or more, four or more, five or more or ten or more, nucleic acid sequences encoding a 5CAR. When the 5CAR T cell comprises two or more sequences encoding a 5CAR, the sequences may encode the same 5CAR or different 5CARs.

A T cell expressing a CD7-specific CAR (7CAR) may comprise one or more, such as two or more, three or more, four or more, five or more or ten or more, nucleic acid sequences encoding a 7CAR. When the 7CAR T cell comprises two or more sequences encoding a 7CAR, the sequences may encode the same 7CAR or different 7CARs.

The 3CAR T cell has completely eliminated expression of one or more of the TCR complex components described above. Accordingly, the 3CAR T cell may have a reduced or completely eliminated capacity to induce off-target effects such as GVHD following administration to a HLA-mismatched recipient or patient.

The 3CAR T cell may have reduced or completely eliminated expression of MHC class I and/or MHC class II. Accordingly, the 3CAR T cell may be subject to minimal amount of rejection when administered to a HLA-mismatched recipient or patient.

### Medical uses

The disclosure provides, for illustrative purposes, a T cell of the disclosure for use in treating disease.

The disclosure also provides, for illustrative purposes, a T cell of the disclosure for use in a method of treating cancer, an autoimmune or auto inflammatory disease, HIV infection, or GVHD, the method comprising administering the T cell to an individual in need thereof.

The cancer may be the cancer a T cell malignancy. The T cell malignancy may be T-cell acute lymphoblastic leukaemia (T-ALL) or T cell lymphoma. The cancer may be primary cancer or secondary cancer.

The autoimmune condition may be alopecia areata, autoimmune encephalomyelitis, autoimmune hemolytic anemia, autoimmune hepatitis, dermatomyositis, diabetes (type 1), autoimmune juvenile idiopathic arthritis, glomerulonephritis, Graves' disease, Guillain-Barre syndrome, idiopathic thrombocytopenic purpura, myasthenia gravis, autoimmune myocarditis, multiple sclerosis, pemphigus/pemphigoid, pernicious anemia, polyarteritis nodosa, polymyositis, primary biliary cirrhosis, psoriasis, rheumatoid arthritis, scleroderma/systemic sclerosis, Sjögren's syndrome, systemic lupus erythematosus, autoimmune thyroiditis, uveitis or vitiligo. The autoinflammatory condition may be Familial Mediterranean fever (FMF), Hyperimmunoglobulinemia D with recurrent fever (HIDS), TNF receptor associated periodic syndrome (TRAPS), Muckle-Wells syndrome (urticaria deafness amyloidosis), Neonatal onset multisystem inflammatory disease (NOMID), or Deficiency of the interleukin-1-receptor antagonist (DIRA). In any of the aspects above, the method may comprise administering an allogeneic transplant to the individual. The allogeneic transplant may be a haematopoietic stem cell transplant, such as a bone marrow, peripheral blood or cord blood transplant.

The disclosure further provides, for illustrative purposes, a T cell of the disclosure for use in a method of depleting host T cells in an individual, the method comprising administering the T cell to the individual. The method is carried out before an allogeneic transplant is administered to the individual. The transplant may be of an organ, a tissue, or cells. In this way, the individual is "conditioned" prior to receiving the transplant. By providing 3CAR T cells targeted against host T cells, host immunity is depleted. Thus, the subsequent transplant is less likely to be rejected.

### Pharmaceutical compositions

For illustrative purposes, the T cells of the disclosure may be provided as a pharmaceutical composition. The pharmaceutical composition preferably comprises a pharmaceutically acceptable carrier or diluent. The pharmaceutical composition may be formulated using any suitable method. Formulation of cells with standard pharmaceutically acceptable carriers and/or excipients may be carried out using routine methods in the pharmaceutical art. The exact nature of a formulation will depend upon several factors including the cells to be administered and the desired route of administration. Suitable types of formulation are fully described in Remington's Pharmaceutical Sciences, 19th Edition, Mack Publishing Company, Eastern Pennsylvania, USA.

The T cells or pharmaceutical composition may be administered by any route. Suitable routes include, but are not limited to, intravenous, intramuscular, intraperitoneal or other appropriate administration routes. The T cells or pharmaceutical composition are preferably administered intravenously.

Compositions may be prepared together with a physiologically acceptable carrier or diluent. Typically, such compositions are prepared as liquid suspensions of cells. The cells may be mixed with an excipient which is pharmaceutically acceptable and compatible with the active ingredient. Suitable excipients are, for example, water, saline, dextrose, glycerol, of the like and combinations thereof.

In addition, if desired, the pharmaceutical compositions of the invention may contain minor amounts of auxiliary substances such as wetting or emulsifying agents, pH buffering agents, and/or adjuvants which enhance effectiveness. The composition preferably comprises human serum albumin.

One suitable carrier or diluents is Plasma-Lyte A^{®}. This is a sterile, nonpyrogenic isotonic solution for intravenous administration. Each 100 mL contains 526 mg of Sodium Chloride, USP (NaCl); 502 mg of Sodium Gluconate (C6H11NaO7); 368 mg of Sodium Acetate Trihydrate, USP (C2H3NaO2•3H2O); 37 mg of Potassium Chloride, USP (KCl); and 30 mg of Magnesium Chloride, USP (MgCl2•6H2O). It contains no antimicrobial agents. The pH is adjusted with sodium hydroxide. The pH is 7.4 (6.5 to 8.0).

The T cells are administered in a manner compatible with the dosage formulation and in such amount will be therapeutically effective. The quantity to be administered depends on the subject to be treated, the disease to be treated, and the capacity of the subject's immune system. Precise amounts of T cells required to be administered may depend on the judgement of the practitioner and may be peculiar to each subject.

Any suitable number of T cells may be administered to a subject. For example, at least, or about, 0.2 × 10⁶, 0.25 × 10⁶, 0.5 × 10⁶, 1.5 × 10⁶, 4.0 × 10⁶ or 5.0 × 10⁶ cells per kg of patient may administered. For example, at least, or about, 10⁵, 10⁶, 10⁷, 10⁸, 10⁹ cells may be administered. As a guide, the number of cells of the invention to be administered may be from 10⁵ to 10⁹, preferably from 10⁶ to 10⁸. Typically, up to 2 × 10⁸ 3CAR T cells are administered to each patient. In such cases where cells are administered or present, culture medium may be present to facilitate the survival of the cells. In some cases the cells of the disclosure may be provided in frozen aliquots and substances such as DMSO may be present to facilitate survival during freezing. Such frozen cells will typically be thawed and infused or placed in a buffer or medium either for maintenance or for administration.

The following Examples illustrate the invention.

### Examples

### Example 1 - Generation of a CD3-specific 3CAR

First, a CD3-specific CAR (3CAR) was derived from the antigen binding elements of the mouse anti-human CD3 monoclonal antibody, OKT3. The variable heavy chain and variable light chain published by Pfishershammer *et al.* were codon optimized (by GeneArt) to generate a single chain variable fragment (scFv). The scFv was linked to activation domains derived from 41BB and CD3ζ. The resultant 3CAR construct was cloned into a lentiviral vector (pCCL) under the control of a PGK promoter using the BamH1 and Mlu1 sites (Figure 1). Vector produced in 293T using a VSV-G envelope yielded excellent titres of 1.1x 10^9 IU/ML.

### Example 2 - Generation of 3CAR T cells

This example demonstrates that disrupting endogenous expression of a TCR complex component in a T cell prior to expressing an introduced nucleic acid encoding a 3CAR to the cell was advantageous to the amplification of 3CAR T cells. In contrast, the traditional approach (where a nucleic acid sequence encoding a 3CAR is introduced and expressed in a T cell prior to disrupting endogenous expression of a TCR complex component) resulted in 'T on T' killing in the cultures.

Figure 2 demonstrates that T cells simply transduced to express 3CAR do not propagate. Here, PBMNCs were transduced with LV-3CAR at an MOI of 5 on day 1 of culture with anti-CD3/CD28 stimulation. The resultant T cells were monitored over 11 days using FAB surface stain (CAR); N=4. The absence of propagation indicates that the T cells are subject to toxicity, which may be due to recognition of endogenous of CD3 as soon as 3CAR is expressed, and/or "T on T" cytotoxicity.

This reduced propagation of CAR-expressing T cells is specific to T cells expressing 3CAR (Figure 3). PBMNC were activated using CD3/28 stimulation and then transduced after 24hrs with LV-3CAR or LV-CAR19 at MOI of 5. On day 7, efficient transduction was detected for control (CAR19) T cells but not 3CAR T cells using stains for FAB (CAR) and CD3.

Furthermore, using the traditional approach to generate 3CAR T cells after having disrupted endogenous expression of a TCR complex component did not produce a sufficient yield of 3CAR T cells for use in a patient (Figure 4). A schematic of the traditional approach is shown in Figure 4a. PBMNCs were activated by anti-CD3/CD28 stimulation. After 2 days, the resultant T cells were transduced with 3CAR of Example 1, cultured at 30°C and subsequently electroporated with TRAC specific Talens at 24h post-transduction. After a further 24h, cells were washed and cultured at 37°C until day 7, when they were stained for FAB and CD3 expression and analysed by flow cytometry. By this method, very low yields of FAB (i.e. 3CAR) positive, CD3 (i.e. TCR complex) negative T cells were obtained (Figure 4b).

In contrast, significantly improved yields of 3CAR positive, TCR negative T cells were obtained when the order of CAR introduction and TCR complex component disruption were reversed (Figure 5). Here, T cells were activated by anti-CD3/CD28 stimulation and electroporated with TRAC specific Talens after two days, cultured at 30°C and subsequently transduced with 3CAR vector or control CD19 CAR at 37C (g). On day 6, cells were stained for viability 7AAD, FAB, CD3, CD4, CD8 and TCR expression; N=2The 3CAR vector yielded 57% FAB (3CAR) positive T cells that were virtually all CD3 (TCR complex) negative, i.e. only CD3-CAR+ cells persist following 3CAR. There was no noticeable yield of 3CAR transduced cells in the absence of TCR/CD3 disruption (b upper panel).

In contrast, control LV CAR19 vector shows persistence of CD3+ CAR+ T cells (b, right panels). Therefore, the 3CAR T cells produced using this method appear to "self-select" and become enriched for 3CAR T cells that do not express CD3 (TCR complex) (b lower middle pane;). Both CD4 and CD8 subsets persisted (c).

Self-selection for 3CAR T cells that do not express CD3 (TCR complex) surprisingly abrogated any need for additional processing (e.g. by CliniMacs) to eliminate residual CD3+ T cells before therapeutic use, as demonstrated in Figure 6. Following TALEN TRAC mRNA exposure, 2 million T cells were transduced on Day 3 (as in Figure 4), and monitored over 13 days for CAR3+ by FAB surface stain. Cells expanded markedly to 100 million cells by day 17. In control samples, around 15-25% TCR+ cells remained after TCR knockout with TRAC specific TALENs. However, in the 3CAR expressing samples, residual TCR+ populations were selectively depleted by 3CAR cells, with a residual populations of <0.5% . The target for residual TCR+ cells after magnetic bead based TCRαβ depletion is <1%. Therefore, no further depletion steps were required. The panels on the right of Figure 6 show PCR sequencing derived molecular signatures of non homologous end joining repair across the TRAC locus in around 70% of alleles. As the gene is expressed by allelic exclusion, this data is consistent with the near complete TCR disruption confirmed by flow analysis. Thus, the newly developed method would provide excellent yields of therapeutically useful cells, and surprisingly without the need for additional processing.

Figure 7 demonstrates how 3CAR T cells are capable of specific effector function against TCR/CD3 leukemic target cells. The left panels show two groups of leukemic Jurkat T cells, with and without TCRαβ expression on their surface. ⁵¹Cr labelled TCR+ve (white symbols) or TCR-ve (black symbols) of these target Jurkat cells were cocultured with either 3CAR+ cells or untransduced control. 3CAR+ cells (but not untransduced cells) specifically mediated high levels of cytotoxicity of target expressing TCR/CD3 (white symbols) but not TCR/CD3 negative targets (Black symbols). Thus, the TCR-depleted 3CAR T cells of the invention are efficiently target TCR/CD3 expressing T cells.

### Example 3 - TCRαβ/CD3 disruption enables CD3-specific anti-leukemic T cell immunotherapy

### Overview

T cells engineered to express chimeric antigen receptors (CARs) against B cell antigens are being investigated as cellular immunotherapies. Similar approaches designed to target T cell malignancies have been hampered by the critical issue of 'T on T' cytotoxicity, whereby fratricide or self-destruction of healthy T cells prohibits cell product manufacture. To date, there have been no reports of T cells engineered to target the definitive T cell marker, CD3. Recent improvments in gene editing now provide access to efficient disruption of such molecules on T cells and this has provided a route to generation of 3CAR, CD3 specific CAR T cells. T cells were transduced with a lentiviral vector incorporating an anti-CD3ε CAR derived from OKT3, either before or after TALEN mediated disruption of the endogenous TCRαβ/CD3 complex. Only transduction after disrupting assembly of TCRαβ/CD3 yeilded viable 3CAR T cells and these cultures were found to undergo self-enrichment for 3CAR+TCR-CD3- T cells without any further processing. Specific cytotoxicity against CD3ε was demonstrated against primary T cells and against childhood acute lymphoblastic leukemia (T-ALL). 3CAR T cells mediated potent anti-leukemic effects in a human:murine chimeric model, supporting the application of cellular immunotherapy strategies against T cell malignancies. 3CAR provides a 'bridging' strategy to achieve T cell eradication and leukemic remission ahead of conditioned allogeneic stem cell transplantation.

### Introduction

There has been notable progress in the development of engineered T cell therapies against B cell malignancies, with chimeric antigen receptors (CARs) against B cell antigens including CD19, CD20, CD22 and other targets have proven highly effective against refractory B cell leukemia [1]. CARs usually comprise an extracellular domain derived from an antibody single-chain variable fragment (scFv), linker, transmembrane moiety and activation domains derived from combinations of CD28, 41BB, and CD3 [2]. Most of these therapies have been generated in a bespoke manner using autologous or HLA-matched allogeneic peripheral blood mononuclear cell harvests, although recently non-matched 'universal' donor CAR19 T cells manufactured using TALEN (Transcription activator-like effector nuclease) gene editing have been reported [3, 4]. The development of similar approaches against T cell malignancies has been extremely challenging, not least because of fratricidal T-on-T cytotoxicity during cell manufacture. Certain cell surface antigens such as CD5 [5] have been targeted using specific CARs and downregulation of their expression in activated T cells has allowed effector cells to expand and yield a product. Similarly, CD4 has been targeted by CD8 T cells expressing a CD4 specific CAR [6] and T cells directed against one or other of the T cell receptor constant regions (TRBC1 or TRBC2) have also been developed and rely on eradicating whichever of the two compartments any leukaemic blasts are derived from [7]. Alternatively, the disruption of the expression of the target ligand in T cells has recently been reported for another T cell receptor, CD7 [8]. In that case, electroporation of CRISPR (Clustered regularly interspaced short palindromic repeat)/Cas9 reagents was used to disrupt CD7 expression in T cells transduced to express anti-CD7 CAR. There was no evidence of detrimental effects on T cell function or cytotoxicity, including in a human murine tumour model.

We report anti-T cell immunotherapy using 3CAR, a chimeric antigen receptor against the CD3ε chain of the T cell receptor complex, generated by fusing the scFv regions of OKT3, a widely used anti-CD3ε monoclonal antibody, with a transmembrane stalk and intracellular activation domains from 41BB and CD3ζ (Figure 8a). Effective manufacturing of anti-CD3 specific T cells was only possible following critically scheduled TALEN mediated disruption of the TCRαβ/CD3 complex; once modified, 3CAR T cells rapidly dominated cultures and self-enriched without the need for further processing to deplete residual TCRαβ T cells required for other gene-edited CAR T cells [5]. Anti-CD3 cellular immunotherapy could deliver valuable anti-leukemic effects in relapsed refractory disease and in the first instance, would be deployed as a form of cellular conditioning to secure molecular remission ahead of allogeneic stem cell transplantation.

### Results and Discussion

T-cell acute lymphoblastic leukemias (T-ALL) account for around 15% of paediatric, and 25% of adult acute lymphoblastic leukemias (ALL), and although outcomes have improved over time, most relapsing subjects face high levels of morbidity and mortality [9]. To date there have been no reports of CAR T cell therapy targeting the definitive T cell marker CD3. Previous attempts to generate anti-CD3 CAR cells been reported using CD3- natural killer (NK) cells, which were then able to specifically eliminate CD3+ lymphoma lines and primary T cell targets in vitro and CD3+ Jurkat leukemia in human:murine chimeras [10]. We generated 3CAR by fusing the scFv fragment of OKT3 (a clinical phase anti-CD3ε monoclonal antibody) to a CD8 transmembrane region linked to 41BB and CD3ζ signalling domains. Lentiviral expression of 3CAR (Figure 8A) in cell lines lacking expression of CD3ε (HEK293T cells) was found to be stable, dose-dependent and saturable at high multiplicities of infection (MOI) (Figure 12). However, in primary CD3+ T cells, lentiviral transduction of T cells activated with anti-CD3/CD28 (TransAct reagent) resulted in inefficient 3CAR gene transfer, with only around 10% modified cells present after 4 days, and none detectable by day 11 (Figure 12) as a result of 'T on T' cytotoxicity. In order to alleviate 3CAR mediated destruction of T cells, gene-editing was employed to disrupt assembly of the multimeric TCRαβ/CD3 complex. We reasoned that disruption and non-homolgous end joining mediated repair of the TRAC locus would not only prevent expression of TCRα, but also disrupt cell surface CD3e expression. First, we attempted to dissemble the TCRαβ/CD3 multimeric complex expression on T cells by electroporation of TRAC-specific TALEN mRNA immediately after activation and lentiviral transduction with 3CAR (Figure 12). This reflected our existing approach for the generation of universal TCRαβ- CAR19+ T cells [3, 4]. This order of transduction followed by editing was known to support high levels of proliferation in T cells, and it was hypothesised that constitutive CAR signalling in TCR- T cells provides resilience and promotes survival through subsequent gene editing steps. Whilst around 80% of cells were disrupted for both CD3 and TCRαβ, the eventual 3CAR T cell yield was poor (Figure 8G, 12) (in contrast to CAR19 vectors) and this suggested widespread targeting of CD3ε antigen leading to self destcruction and/or rapid fratricide of other CD3+ T cells in the culture.

Next, the event sequence was reversed to first deplete endogenous CD3ε expression in activated T cells, and then undertake LV transduction within an 18-24hour time window (Figure 8B, 13).Here, within 7 days of initiation, 57% of T cells expressed 3CAR (Figure 8C) with approximately equal proportions of CD4+ and CD8+ T cells (Figure 8D), and comparable outcomes to vectors expressing CAR19 (Figure 8E, F). Importantly, the cultures 'self enriched' for T cells devoid of CD3/TCRαβ, obviating the need for any further processing to deplete residual TCR+ T cells which comprised <0.2% of the product. In contrast, residual TCR+ populations in TCR-CAR19+ cell products required removal by bead mediated depletion by targeting TCRαβ to achieve a target of <1%TCR+ cells in the final product. This is a stringent threshold that strongly influences allogeneic dosing strategies where GVHD is avoided by capping TCRαβ carriage to <5×10⁴/kg.

To assess scalability, cells were activated, edited and transduced and 2×10⁶ 3CAR T cells cultured further in a gas-permeable static cell culture flask (G-Rex). Cells expanded by 100 fold over 11 days (Figure 9A) and were devoid of TCR/CD3 expressing cells with residual populations of <0.5% (Figure 9B). After electroporation of TRAC mRNA, CD3+TCR+T cells were selectively depleted in 3CAR T cell cultures (Figure 9B). On phenotype analysis, ≤5% of cells expressed the T cell exhaustion marker PD-1, and residual B cells (CD20) or NK cells (CD56) were negligible (Figure 14). TIDE-PCR sequencing across the TRAC locus confirmed an allele modification frequency of 70% based on molecular signatures of non-homologous end joining repair (Figure 9C). As the TRAC locus exhibits allelic exclusion, this [11] frequency of modification was consistent with the near complete TCR disruption assessed by flow analysis.

3CAR T cells mediated specific cytotoxicity against CD3+ leukemic targets. This was assessed in vitro using multiple approaches. Firstly, 51Cr- labelled TCR/CD3+ or TCR/CD3- Jurkat leukemia cells [12] were cocultured with either 3CAR T cells or non-transduced control T cells. 3CAR+ cells mediated specific high level cytotoxicity of targets expressing TCR/CD3+, but not TCR/CD3- targets (Figures 9D, 14).Next a single-cell flow-based assay also confirmed that, 3CAR+ cells mediated highly specific cytotoxicity of leukemic TCR/CD3+ targets but not TCR/CD3- targets. EGFP expressing TCR/CD3+ Jurkat cells were almost completely eliminated by 3CAR cells (0.8%), compared with untransduced effector controls (61%). TCR/CD3- leukemia cells were unaffacted by 3CAR (57%) or effector controls (54%) (Figure 9E, F). 3CAR T cells also exhibited interferon (IFN)-y production responses against TCR/CD3+ leukemic Jurkat targets (Figure 15).

3CAR T cells also mediated cytotoxic effects against primary CD3+ T cells purified from healthy donor peripheral blood mononuclear cells (PBMC) and loaded with CSFE fluorescent dye. Only around 4% of these cells survived compared to 42% in control cultures. TCR/CD3- populations were not eliminated by 3CAR cells, confirming specificity against CD3ε (Figure 10A,B 16). 3CAR T cellscultured with allogeneic PBMCs or purified allogeneic TCR/CD3+ T cells exhibited cytokine interferon (IFN)-y responses and low level secretion of tumour necrosis factor (TNF)-α, IL-10, IL-6 and IL-4 (Figures 10C, 15). 3CAR T cells also exhibited notable [3H]-thymidine proliferation responses when cocultured with irradiated allogenic CD3+ T cells. Proliferation against irradiated allogeneic CD3-depleted PBMC was reduced, but control T cell mediated allo-recognition was intact Figure 10D). These data suggest 3CAR would mediate elimination of healthy TCRαβ/CD3 T cells, an effect that could be harnessed during conditioning procedures ahead of allogeneic transplantation.

To further evaluate the function of 3CAR T cells cultures were established against viable tissue bank samples from children with T cell acute lymphoblastic leukemia (T-ALL). Flow cytometry verified CD3, CD7, CD19 and CD34 expression upon thawing in these targets (Figure 22). In the case of T-ALL#1 cells, 86% CD3 expressing cells were almost completed eliminated by 3CAR+ cells (Figure 10E). Despite the phenotypic heterogeneity of the T-ALL, in 6/6 samples, 3CAR cells displayed highly specific cytotoxicity against CD3+ but not CD3-CD19+ cells (Figures 10F,17, 18).

In vivo functionality of 3CAR T cells was assessed in a humanized murine model of leukemic T cell clearance. NSG mice were inoculated intravenously with either 10×10⁶ TCR/CD3+eGFP+LUC+ or TCR/CD3- eGFP+LUC+ Jurkat T cell targets and imaged after 3 days to confirm leukemia establishment, and were then injected with effector cells. These comprised either 3CAR or untransduced effector T cells. Serial bioluminescence imaging on days 3-18 showed rapid clearance of TCR/CD3+ Jurkats in the cohort receiving 3CAR T cells with negligible signal detected by day 11, in contrast to mice receiving non-transduced T cells or PBS where leukemia progressed (Figure 11 A,B). By day 18, disease burden had increased by >60-fold in animals receiving untransduced effectors compared to the 3CAR effector group (Figure 11 C). As expected, neither 3CAR effectors nor control T cells exhibited anti-leukemic effect against TCR/CD3- Jurkats lacking target antigen expression (Figure 19). At necroscopy, flow cytometry was used to detect EGFP+ Jurkat T cells and/or CD45+CD2+GFP- effector T cell populations in bone marrow (Figure 11D). Analysis from day 18 samples found complete clearance of TCR/CD3+ Jurkat T cells in 5/5 animals in 3CAR treated animals compared with control animals receiving PBS or untransduced T cells (Figures 11 E, 19 A). In most animals only very small numbers of TCR-GFP+ Jurkats cells were identified, except one animal where there appeared to have been an outgrowth of CD3-GFP+ Jurkats that had presumably not been to susceptible to 3CAR (Figures 11, 20). 3CAR effectors retained expression of their chimeric receptor and CD4+/CD4- marking at levels comparable to those in the original inoculum (Figures 11F, 20, 21). Although, 3CAR expression (24%) was detected in animals engrafted with CD3-TCR- Jurkats, absence of suitable target antigen resulted in rapid accumulation of disease in these animals (Figure 20).

The gene editing platform used in this iteration of 3CAR was based on TALENs, and derived from our experience with similar reagents. Previously we have reported minimal evidence of off-target effects using high throughput NGS interrogation of in silico predicted sites of TALEN activity. Alternative editing platforms may be equally effective, although are likely to need to operate within similarly well defined window of activity to ensure target antigen removal before 3CAR expression.

The inventors have found that gene editing to disrupt TCR expression can be exploited to enable 3CAR T cells to be generated through avoidance of 'T on T' fratricide or self destruction. In previous campaigns the inventors had found that the generation of universal TCR-CAR19+ T cells was most efficient when TRAC disruption was performed after lentiviral transduction. However, a similar schedule for 3CAR resulted in poor yields, presumably because prior expression of 3CAR mediated detection of CD3ε. A strictly ordered sequence of TRAC disruption ahead of 3CAR expression was found to be essential for the generation of 3CAR, and this event precedence was incorporated into scaled production of effector cells. The resulting 3CAR cells were potent mediators of T cell specific cytotoxity, both against primary T cells in vitro and against human leukemia in chimeric models.

The expression of CD3 on leukemic T cells can be variable but critically 3CAR mediated cytotoxicity of CD3+ targets was near complete in 6/6 samples evaluated. It is likely, that for effective elimination of heterogenous leukemias, a strategy targeting multiple T cell antigens will be required, akin to combinational antibiotic therapy for mycobacterial disease or antiviral therapy against HIV. Based on our analysis, a combination of CARs targeting CD3 and CD7 could be highly effective against childhood T-ALL. Applications in patients with CD3+ ALL who are refractory to conventional chemotherapy is envisaged, most likely as a 'bridge to transplant' after achieving molecular remission. In this setting, pre-manufactured 3CAR T cells from a healthy allogeneic donor could be used in an 'off the shelf manner, similar to 'universal' CAR19 T cells against B cell malignancies. However, whereas suppression of the normal B cell compartment by persisting CAR T cells can be addressed by administration of replacement immunoglobulin therapy, long-term T cell lymphopenia mediated by 3CAR could be prohibitively immunosuppressive. Once remission was secured, additional conditioning and serotherapy would be used to eradicate 3CAR, a step essential for subsequent donor derived T cell reconstitution to proceed. Such a strategy could also lead to broader 3CAR mediated conditioning strategies for allogeneic stem cell transplantation providing an alternative to intensive chemotherapy regimens.

### Methods

**Cell** lines: Clonal expansion of TCR/CD3+ and TCR/CD3- expressing Jurkats cells (Acute T Cell Leukemic cell line) maintained in culture in RPMI-1640 (Gibco-BRL, San Francisco, CA, USA) supplemented with10% fetal bovine serum (Sigma-Aldrich).

**Generation of anti-CD3 CAR:** CD3-specific CAR (3CAR) was derived by codon optimisation (geneart) of variable heavy chain and variable light chain antigen binding elements of the mouse anti-human CD3 monoclonal antibody, OKT3. (Pfishershammer et al). The scFv was linked to activation domains derived from 41BB and CD3ζ as previously described. The resultant 3CAR construct was cloned into a lentiviral vector (pCCL) under the control of a PGK promoter (Figure 1). Vector stocks pseudotyped with a VSV-G envelope were generated in 293T cells and yielded titres >1.0× 10⁹ IU/ML

**Generation of CAR-modified T cells:** Peripheral blood mononuclear cells were obtained from healthy donors and were cultured in 48-well plates at a density of 1 × 10⁶/mL in TexMACS (Miltenyi Biotec), 3% Human Serum (Seralab) + 20 ng/mL Human Recombinant interleukin (IL)-2 (Miltenyi Biotec) and activated with TransAct Reagent (Miltenyi Biotec). Activated T cells were electroporated with TALEN_TCR_2a Right and Left mRNA (TRAC mRNA) {Berdien, 2014 #461} (TriLink Biotechnologies) on day 2, cells were divided into untransduced controls or transduced with CD3-CAR vector at a MOI of 5.

**Flow cytometry:** Cells were stained with the following primary anti-human antibodies CD2, CD3, TCRαβ, CD4, CD8, CD19, CD20, CD56 and programmed death 1 (PD-1) (from Miltenyi Biotec) and mCD11b. To assess the efficiency of CD3-CAR transduction, cells were stained using a Biotin-SP (long spacer) AffiniPure F(ab') Fragment Goat AntiMouse immunoglobulin (Ig)G, F(ab') Fragment Specific antibody (Jackson Immunoresearch, Stratech Scientific Limited, Suffolk, UK) followed by Streptavidin-APC or Streptavidin-Fluorescein isothicyanate (FITC) (Biolegend). Cells were acquired on a Cyan or on a BD LSRII (BD Biosciences, Oxford, UK) and analysis performed using FlowJo v10 (TreeStar Inc. Ashland OR, USA).

**Chromium release assay of cytotoxicity:** Cytotoxic activity of 3CAR cells was assessed by chromium 51 (⁵¹Cr) release assay. For this 5 × 10³ 51Cr-labeled TCR/CD3-positive Jurkats or TCR/CD3-negative Jurkats target cells were incubated with 3CAR or untransduced control effector cells at increasing effector-to-target (E:T) ratios in 96-well microplates for 4 h at 37°C. ⁵¹Cr release was then measured in a microplate scintillation counter (Wallac 1450 MicroBeta TriLux).

**Flow based cytotoxicty assay:** CD3 CART cells or control non-transduced T cells were cocultured with TCR/CD3-positive Jurkats or TCR/CD3-negative Jurkats target cells that were loaded with CFSE dye (Cell Trace, Invitrogen), at the effector to target ratio 1:1(100,000 of both effector and target cells) for 24h. Dot plots show representative frequency of gated CFSE+ tumor cells at the end of coculture. CD3 CART cells or control non-transduced T cells also were cocultured with healthy donor PBMC, CD3+ or CD3-cells (MicroBeads, Miltenyi, Bergisch-Gladbach, Germany).

**Cytokine production:** 3CAR-transduced cells were thawed, washed and re-suspended in RPMI 10% FCS at a concentration of 1 × 10⁶/mL. TCR/CD3-negative Jurkat cells, TCR/CD3- positive Jurkat cells and healthy donor PBMCs were all counted and also adjusted to a concentration of 1 × 10⁶/mL. Cells were mixed at a 1:1 ratio in 24-well tissue culture plates and incubated at 37°C. After 24-h supernatants were harvested and stored at -80°C. Levels of cytokine in the stored supernatants were quantified using the TH1/TH2/TH17 cytometric bead array kit (CBA; BD Biosciences) as per the manufacturer's protocol.

**Mixed leukocyte reaction and ³H-thymidine incorporation:** To assess allogeneic T-cell proliferation in a MLR, 3CAR T cells were mixed with target PBMCs or PBMCs depleted of CD3 using CD3 MicroBeads, (Miltenyi, Bergisch-Gladbach, Germany), in RPMI 10% FCS at a 1:1 ratio in 96-well plates. To asses a one-way MLR, targets cells were irradiated (3,000 rads) prior to coculture. Targets alone were used as negative controls, and all cultures were run in triplicate. After co-incubation for 5 days, ³H-thymidine (1 µCi per well) was added for 18 h before harvesting onto filter mats for radioactivity measurement in a scintillation counter (Wallac 1450 MicroBeta TriLux).

**In vivo anti-tumor activity:** NOD/SCID/c-/- (NSG) mice, were inoculated IV with 10×10⁶ TCR+ve or TCR-ve Jurkat T cell tumour targets by tail vein injection on day 0. The Jurkat T cell targets had been stably transduced to express both enhanced green fluorescent protein (EGFP) and Luciferase and following TALEN-mediated TCRαβ disruption had been sorted for TCR+ and TCR- expressers. Tumour engraftment was confirmed by in vivo imaging of bioluminescence using an IVIS Lumina III In Vivo Imaging System (PerkinElmer, Massachusetts, USA, live image version 4.5.18147) on day 3. TCR+ or TCR- injected mice were further injected on day 4 with either PBS (n=2), 10×10⁶ untransduced T cells (n=4) or 10×10⁶ 3CAR T cells (n=6) per tumour group. Analysis of tumor clearance was performed by serial bioluminescent imaging on days 3, 7, 11,and 18 and processing of bone marrow for the monitoring of tumor progression vs clearance was carried out on day 18. Bone marrow samples were processed by a red blood cell lysis followed by staining for flow cytometry. All animal studies were approved by the University College London Biological Services Ethical Review Committee and licensed under the Animals (Scientific Procedures) Act 1986 (Home Office, London, United Kingdom).

**Primary T-ALL blasts:** Primary T-ALL patient samples are all from the ALL2003 trial where peripheral blood was often sent at diagnosis instead of bone marrow, with information on CD3+ status, and other surface antigens (see table) (From Bloodwise Childhood Leukaemia Cell Bank). To phenotype patient cells, cells were stained with the following primary antibodies CD3, CD7, CD19 and CD34. For FACS-Based Killing assay, CD3 CART cells, control CAR19 T cells or control non-transduced T cells were cocultured with T-ALL patient sample target cells that were loaded with CFSE dye (Cell Trace, Invitrogen), at the effector to target ratio 1:1(100,000 of both effector and target cells) for 24h.

### Statistics

For in vitro studies, data points from individual donors are shown in all figures. Statistical significance in pairwise comparisons was determined by unpaired two-tailed Student t test. For in vivo studies, a two-tailed Man-Whitney U test was used for non-parametric comparison of grouped data and values are presented as mean percentages of three or more samples with standard error of the mean (SEM) or standard deviation (SD) or as a median with the 25th and 75th percentiles stated. Linear Regression was used for the comparison of serial measurements, taking each Y value as an individual point. All statistical analysis was performed using GraphPad Prism software version 5.01.

### References

1. Riviere, I. and M. Sadelain, Chimeric Antigen Receptors: A Cell and Gene Therapy Perspective. Mol Ther, 2017. 25(5): p. 1117-1124.
2. Sadelain, M., R. Brentjens, and I. Riviere, The basic principles of chimeric antigen receptor design. Cancer Discov, 2013. 3(4): p. 388-98.
3. Poirot, L., et al., Multiplex Genome-Edited T-cell Manufacturing Platform for "Off-the-Shelf" Adoptive T-cell Immunotherapies. Cancer Res, 2015. 75(18): p. 3853-64.
4. Qasim, W., et al., Molecular remission of infant B-ALL after infusion of universal TALEN gene-edited CAR T cells. Sci Transl Med, 2017. 9(374).
5. Mamonkin, M., et al., A T-cell-directed chimeric antigen receptor for the selective treatment of T-cell malignancies. Blood, 2015. 126(8): p. 983-92.
6. Pinz, K., et al., Preclinical targeting of human T-cell malignancies using CD4-specific chimeric antigen receptor (CAR)-engineered T cells. Leukemia, 2016. 30(3): p. 701-7.
7. Maciocia, P.M., et al., Targeting the T cell receptor beta-chain constant region for immunotherapy of T cell malignancies. Nat Med, 2017.
8. Gomes-Silva, D., et al., CD7-edited T cells expressing a CD7-specific CAR for the therapy of T-cell malignancies. Blood, 2017. 130(3): p. 285-296.
9. Goldberg, J.M., et al., Childhood T-cell acute lymphoblastic leukemia: the Dana-Farber Cancer Institute acute lymphoblastic leukemia consortium experience. J Clin Oncol, 2003. 21(19): p. 3616-22.
10. Chen, K.H., et al., Novel anti-CD3 chimeric antigen receptor targeting of aggressive T cell malignancies. Oncotarget, 2016. 7(35): p. 56219-56232.
11. Alam, S.M., I.N. Crispe, and N.R. Gascoigne, Allelic exclusion of mouse T cell receptor alpha chains occurs at the time of thymocyte TCR up-regulation. Immunity, 1995. 3(4): p. 449-58.
12. Schneider, U., H.U. Schwenk, and G. Bornkamm, Characterization of EBV-genome negative "null" and "T" cell lines derived from children with acute lymphoblastic leukemia and leukemic transformed non-Hodgkin lymphoma. Int J Cancer, 1977. 19(5): p. 621-6.

## Claims

1. An in vitro method for producing a T cell expressing one or more chimeric antigen receptors (CARs) specific for CD3, comprising (i) eliminating endogenous expression of a T cell receptor (TCR)/CD3 complex component in a T cell, (ii) introducing a nucleic acid sequence encoding the one or more CARs to said T cell of (i), and subsequently (iii) expressing the one or more CARs of (ii) in said T cell of (i); wherein (i) is carried out 10 to 40 hours before (ii).

2. The method of claim 1, wherein (i) is carried out 20 to 30 hours before (ii).

3. The method of claim 1 or 2, wherein:
(a) the TCR/CD3 complex component is TCR alpha, TCR beta, TCR gamma, TCR delta or any chain of the CD3 complex; and/or:
(b) the endogenous expression of the TCR/CD3 complex component is eliminated in (i) using a gene editing technique, optionally wherein the gene editing technique is ZFNs, Meganuclease, Mega-TALENs, TALENs, or CRISPR.

4. The method of claim 3 , wherein the gene editing technique disrupts:
(a) the TRAC locus or a region controlling its expression;
(b) the TCR beta locus or a region controlling its expression;
(c) TCR gamma locus or a region controlling its expression;
(d) TCR delta locus or a region controlling its expression; or
(e) a gene locus for a CD3 chain or a region controlling its expression; optionally wherein the CD3 chain is CD3y, CD3δ, CD3ε or CD3ζ.

5. The method of any one of the preceding claims, wherein the CAR comprises an scFV from a CD3-specific antibody, optionally wherein the CD3-specific antibody is OKT3, RIV-9, UCHT1, SK7, MEM57, MEM92, HIT3A, or an antibody that competes for CD3 binding with the OKT3 antibody.

6. The method of any one of the preceding claims, wherein:
(a) the CAR comprises a 4-1BB activation domain;
(b) the CAR comprises a CD3ζ activation domain;
(c) the T cell is a cord blood T cell;
(d) the T cell is a CD4+ T cell, a CD8+ T cell, optionally wherein the T cell is a naive, T helper, effector, memory, effector memory, central memory, memory stem or regulatory T cell; and/or
(e) the T cell is autologous with respect to a patient into which it is to be delivered, or is allogeneic with respect to a patient into which it is to be delivered.

7. The method of any one of the preceding claims, wherein:
(a) the nucleic acid sequence encoding the CAR is introduced to the T cell using a vector;
(b) the method further comprises (iv) introducing a nucleic acid sequence encoding a suicide gene to the T cell;
(c) the method further comprises (iv) introducing a nucleic acid sequence encoding a suicide gene to the T cell, wherein (I) the suicide gene is herpes simplex virus thymidine kinase or an inducible caspase, or (II) the suicide gene encodes an epitope recognised by a monoclonal antibody specific for HerB2, EGFR or CD20, optionally wherein the nucleic acid sequence encoding the suicide gene is introduced to the T cell using a vector, and further optionally wherein
(A) the nucleic acid sequence encoding the CAR and the nucleic acid sequence encoding the suicide gene are introduced to the T cell using the same vector;
(B) the nucleic acid sequence encoding the CAR and the nucleic acid sequence encoding the suicide gene are introduced to the T cell using the same vector, and the nucleic sequence encoding the suicide gene is linked to the nucleic acid sequence encoding the CAR by an IRES element or a nucleic acid sequence encoding a self-cleaving peptide; or
(C) the nucleic acid sequence encoding the CAR and the nucleic acid sequence encoding the suicide gene are introduced to the T cell using the same vector, and the nucleic sequence encoding the suicide gene is linked to the nucleic acid sequence encoding the CAR such that the suicide gene and the CAR are expressed as an in-frame fusion construct.

8. The method of claim 7, wherein the vector is a viral vector; optionally wherein the viral vector is a lentiviral vector, gamma-retroviral vector, alpha-retroviral vector, Sleeping Beauty transposon system or PiggyBac transposon system.

9. The method of any of one of the preceding claims, wherein:
(a) (i) further comprises eliminating endogenous expression of CD52, MHC, and/or a molecule involved in a checkpoint pathway;
(b) following (iii), said T cell exhibits antigen specificity conferred by the specificity of the CAR; optionally wherein said T cell exhibits said antigen specificity 6-24 hours after (ii);
(c) after (iii), formulating the T cell for administration to an individual; optionally wherein formulating the T cell provides a formulation comprising isotonic phosphate buffered saline, EDTA, DMSO and human albumin serum; or wherein the formulation comprises 7.5% w/v DMSO and/or 4% w/v human albumin serum; and/or
(d) after (iii), the method comprises cryopreserving the T cell.

## Patentansprüche

1. In-vitro-Verfahren zur Herstellung einer T-Zelle, die einen oder mehrere für CD3 spezifische chimäre Antigenrezeptoren (CARs) exprimiert, wobei das Verfahren Folgendes umfasst: (i) Eliminieren der endogenen Expression einer T-Zell-Rezeptor- (T Cell Receptor, TCR)/CD3-Komplexkomponente in einer T-Zelle, (ii) Einführen einer Nukleinsäuresequenz, die für den einen oder die mehreren CARs codiert, in die T-Zelle aus (i), und anschließend (iii) Exprimieren des einen oder der mehreren CARs von (ii) in der T-Zelle von (i); wobei (i) 10 bis 40 Stunden vor (ii) durchgeführt wird.

2. Verfahren nach Anspruch 1, wobei (i) 20 bis 30 Stunden vor (ii) durchgeführt wird.

3. Verfahren nach Anspruch 1 oder 2, wobei:
(a) die TCR/CD3-Komplexkomponente TCR alpha, TCR beta, TCR gamma, TCR delta oder eine beliebige Kette des CD3-Komplexes ist; und/oder:
(b) die endogene Expression der Komponente des TCR/CD3-Komplexes in (i) durch eine Gen-Editierungstechnik eliminiert wird, wobei es sich bei der Gen-Editierungstechnik optional um ZFNs, Meganuclease, Mega-TALENs, TALENs oder CRISPR handelt.

4. Verfahren nach Anspruch 3, wobei die Gen-Editierungstechnik Folgendes unterbricht:
(a) den TRAC-Locus oder eine Region, die seine Expression kontrolliert;
(b) den TCR-beta-Locus oder eine Region, die seine Expression kontrolliert;
(c) den TCR-gamma-Locus oder eine Region, die seine Expression kontrolliert;
(d) TCR-delta-Locus oder eine Region, die seine Expression kontrolliert, oder
(e) einen Genlocus für eine CD3-Kette oder eine Region, die seine Expression kontrolliert; wobei die CD3-Kette optional CD3y, CD3δ, CD3ε oder CD3ζ ist.

5. Verfahren nach einem der vorhergehenden Ansprüche, wobei der CAR ein scFV aus einem CD3-spezifischen Antikörper umfasst, wobei der CD3-spezifische Antikörper optional OKT3, RIV-9, UCHT1, SK7, MEM57, MEM92, HIT3A oder ein Antikörper ist, der mit dem OKT3-Antikörper um die CD3-Bindung konkurriert.

6. Verfahren nach einem der vorhergehenden Ansprüche, wobei:
(a) der CAR eine 4-1BB-Aktivierungsdomäne umfasst;
(b) das CAR eine CD3ζ-Aktivierungsdomäne umfasst;
(c) die T-Zelle eine T-Zelle aus Nabelschnurblut ist;
(d) die T-Zelle eine CD4+ T-Zelle, eine CD8+ T-Zelle ist, wobei es sich bei der T-Zelle optional um eine naive, T-Helfer-, Effektor-, Gedächtnis-, Effektorgedächtnis-, zentrale Gedächtnis-, Gedächtnisstamm- oder regulatorische T-Zelle handelt; und/oder
(e) die T-Zelle autolog in Bezug auf einen Patienten ist, an den sie abgegeben werden soll, oder sie allogen in Bezug auf einen Patienten ist, an den sie abgegeben werden soll.

7. Verfahren nach einem der vorhergehenden Ansprüche, wobei:
(a) die Nukleinsäuresequenz, die für den CAR codiert, unter Verwendung eines Vektors in die T-Zelle eingeführt wird;
(b) das Verfahren ferner (iv) das Einführen einer Nukleinsäuresequenz, die für ein Suizidgen codiert, in die T-Zelle umfasst;
(c) das Verfahren ferner (iv) das Einführen einer Nukleinsäuresequenz, die für ein Suizidgen codiert, in die T-Zelle umfasst, wobei (I) das Suizidgen eine Thymidinkinase des Herpes Simplex Virus oder eine induzierbare Caspase ist oder (II) das Suizidgen für ein Epitop codiert, das von einem monoklonalen Antikörper erfasst wird, der spezifisch für HerB2, EGFR oder CD20 ist, wobei die Nukleinsäuresequenz, die für das Suizidgen codiert, optional unter Verwendung eines Vektors in die T-Zelle eingeführt wird, und ferner, wobei optional:
(A) die Nukleinsäuresequenz, die für den CAR codiert, und die Nukleinsäuresequenz, die für das Suizidgen codiert, unter Verwendung desselben Vektors in die T-Zelle eingeführt werden;
(B) die Nukleinsäuresequenz, die für den CAR codiert, und die Nukleinsäuresequenz, die für das Suizidgen codiert, unter Verwendung desselben Vektors in die T-Zelle eingeführt werden, und die Nukleinsäuresequenz, die für das Suizidgen codiert, mit der Nukleinsäuresequenz, die für das CAR codiert, durch ein IRES-Element oder eine Nukleinsäuresequenz, die für ein selbstspaltendes Peptid codiert, verbunden ist; oder
(C) die Nukleinsäuresequenz, die für den CAR codiert, und die Nukleinsäuresequenz, die für das Suizidgen codiert, unter Verwendung desselben Vektors in die T-Zelle eingeführt werden, und die Nukleinsäuresequenz, die für das Suizidgen codiert, mit der Nukleinsäuresequenz, die für den CAR codiert, verbunden ist, so dass das Suizidgen und der CAR als In-Frame-Fusionskonstrukt exprimiert werden.

8. Verfahren nach Anspruch 7, wobei der Vektor ein viraler Vektor ist; wobei der virale Vektor optional ein lentiviraler Vektor, ein gamma-retroviraler Vektor, ein alpharetroviraler Vektor, ein Sleeping Beauty Transposon System oder ein PiggyBac Transposon System ist.

9. Verfahren nach einem der vorhergehenden Ansprüche, wobei:
(a) (i) ferner die Eliminierung der endogenen Expression von CD52, MHC und/oder eines Moleküls, das an einem Checkpoint-Weg beteiligt ist, umfasst;
(b) nach (iii) die T-Zelle eine Antigenspezifität aufweist, die durch die Spezifität des CAR vermittelt wird; wobei die T-Zelle optional die Antigenspezifität 6 bis 24 Stunden nach (ii) aufweist;
(c) nach (iii), Formulieren der T-Zelle zur Verabreichung an ein Individuum; wobei das Formulieren der T-Zelle optional eine Formulierung bereitstellt, die isotonische phosphatgepufferte Kochsalzlösung, EDTA, DMSO und humanes Albuminserum umfasst; oder wobei die Formulierung 7,5 % Gew./Vol. DMSO und/oder 4 % Gew./Vol. menschliches Albuminserum umfasst; und/oder
(d) nach (iii) das Verfahren die Kryokonservierung der T-Zelle umfasst.

## Revendications

1. Procédé in vitro de production d'une cellule T exprimant un ou plusieurs récepteurs antigéniques chimériques (CAR) spécifiques de CD3, comprenant (i) l'élimination de l'expression endogène d'un composant du complexe récepteur de cellule T (TCR)/CD3 dans une cellule T, (ii) l'introduction d'une séquence d'acide nucléique codant pour l'un ou plusieurs CAR dans ladite cellule T de (i), puis (iii) l'expression de l'un ou plusieurs CAR de (ii) dans ladite cellule T de (i) ; dans lequel (i) est effectuée 10 à 40 heures avant (ii).

2. Procédé selon la revendication 1, dans lequel (i) est effectuée 20 à 30 heures avant (ii).

3. Procédé selon la revendication 1 ou 2, dans lequel :
(a) le composant du complexe TCR/CD3 est TCR alpha, TCR bêta, TCR gamma, TCR delta ou n'importe quelle chaîne du complexe CD3 ; et/ou :
(b) l'expression endogène du composant complexe TCR/CD3 est éliminée en (i) en utilisant une technique d'édition de gène, éventuellement dans laquelle la technique d'édition de gène est ZFNs, Méganucléase, Mega-TALENs, TALENs, ou CRISPR.

4. Procédé selon la revendication 3, dans lequel la technique d'édition de gènes perturbe :
(a) le locus TRAC ou une région contrôlant son expression ;
(b) le locus bêta du TCR ou une région contrôlant son expression ;
(c) le locus gamma du TCR ou une région contrôlant son expression ;
(d) le locus delta du TCR ou une région contrôlant son expression ; ou
(e) un locus génique pour une chaîne CD3 ou une région contrôlant son expression ; éventuellement dans lequel la chaîne CD3 est CD3y, CD3δ, CD3ε ou CD3ζ.

5. Procédé selon l'une quelconque des revendications précédentes, dans lequel le CAR comprend un scFV d'un anticorps spécifique de CD3, éventuellement dans lequel l'anticorps spécifique de CD3 est OKT3, RIV-9, UCHT1, SK7, MEM57, MEM92, HIT3A, ou un anticorps qui entre en compétition pour la liaison CD3 avec l'anticorps OKT3.

6. Procédé selon l'une quelconque des revendications précédentes, dans lequel :
(a) le CAR comprend un domaine d'activation 4-1BB ;
(b) le CAR comprend un domaine d'activation CD3ζ ;
(c) la cellule T est une cellule T du sang de cordon ;
(d) la cellule T est une cellule T CD4+, une cellule T CD8+, éventuellement dans laquelle la cellule T est une cellule T naïve, auxiliaire, effectrice, mémoire, effectrice mémoire, mémoire centrale, mémoire souche ou régulatrice ; et/ou
(e) la cellule T est autologue vis-à-vis d'un patient auquel elle doit être administrée, ou est allogénique vis-à-vis d'un patient auquel elle doit être administrée.

7. Procédé selon l'une quelconque des revendications précédentes, dans lequel :
(a) la séquence d'acide nucléique codant pour le CAR est introduite dans la cellule T en utilisant un vecteur ;
(b) le procédé comprend en outre (iv) l'introduction d'une séquence d'acide nucléique codant pour un gène suicide dans la cellule T ;
(c) le procédé comprend en outre (iv) l'introduction d'une séquence d'acide nucléique codant pour un gène suicide dans la cellule T, dans lequel (I) le gène suicide est la thymidine kinase du virus de l'herpès simplex ou une caspase inductible, ou (II) le gène suicide code pour un épitope reconnu par un anticorps monoclonal spécifique de HerB2, EGFR ou CD20, éventuellement dans lequel la séquence d'acide nucléique codant pour le gène suicide est introduite dans la cellule T en utilisant un vecteur, et en outre éventuellement dans lequel
(A) la séquence d'acide nucléique codant pour le CAR et la séquence d'acide nucléique codant pour le gène suicide sont introduites dans la cellule T en utilisant le même vecteur ;
(B) la séquence d'acide nucléique codant pour le CAR et la séquence d'acide nucléique codant pour le gène suicide sont introduites dans la cellule T en utilisant le même vecteur, et la séquence nucléique codant pour le gène suicide est liée à la séquence d'acide nucléique codant pour le CAR par un élément IRES ou une séquence d'acide nucléique codant pour un peptide auto-clivant ; ou
(C) la séquence d'acide nucléique codant pour le CAR et la séquence d'acide nucléique codant pour le gène suicide sont introduites dans la cellule T en utilisant le même vecteur, et la séquence nucléique codant pour le gène suicide est liée à la séquence d'acide nucléique codant pour le CAR de telle sorte que le gène suicide et le CAR sont exprimés sous la forme d'une construction de fusion dans le cadre.

8. Procédé selon la revendication 7, dans lequel le vecteur est un vecteur viral ; éventuellement dans lequel le vecteur viral est un vecteur lentiviral, un vecteur gamma-rétroviral, un vecteur alpha-rétroviral, un système de transposon Sleeping Beauty ou un système de transposon PiggyBac.

9. Procédé selon l'une quelconque des revendications précédentes, dans lequel :
(a) (i) il comprend en outre l'élimination de l'expression endogène de CD52, du CMH et/ou d'une molécule impliquée dans une voie de point de contrôle ;
(b) suivant (iii), ladite cellule T présente une spécificité antigénique conférée par la spécificité du CAR ; éventuellement dans lequel ladite cellule T présente ladite spécificité antigénique 6 à 24 heures après (ii) ;
(c) après (iii), la formulation de la cellule T pour administration à un individu ; éventuellement dans lequel la formulation de la cellule T fournit une formulation comprenant une solution saline tamponnée au phosphate isotonique, de l'EDTA, du DMSO et du sérum d'albumine humaine ; ou dans lequel la formulation comprend 7,5 % p/v de DMSO et/ou 4 % p/v de sérum d'albumine humaine ; et/ou
(d) après (iii), le procédé comprend la cryoconservation de la cellule T.
